# EUROPEAN PATENT SPECIFICATION

(11) **EP 3 068 889 B1**
(45) Date of publication and mention of the grant of the patent: **17.04.2019**
(21) Application number: 14781103.8
(22) Date of filing: 25.09.2014
(51) Int. Cl.: C12N 15/86, A61K 48/00

(54) **GENE THERAPY COMPOSITIONS FOR USE IN THE PREVENTION AND/OR TREATMENT OF NON-ALCOHOLIC FATTY LIVER DISEASE**
GENTHERAPIEZUSAMMENSETZUNGEN ZUR VERWENDUNG BEI DER VORBEUGUNG UND/ODER BEHANDLUNG VON NICHT ALKOHOLBEDINGTER FETTLEBER
COMPOSITIONS DE THÉRAPIE GÉNIQUE DESTINÉES À ÊTRE UTILISÉES DANS LA PRÉVENTION ET/OU LE TRAITEMENT D'UNE STÉATOSE HÉPATIQUE NON-ALCOOLIQUE

(30) Priority: 26.09.2013 EP 13382369
(43) Date of publication of application: 21.09.2016
(73) Proprietor: Universitat Autònoma de Barcelona, 08193 Cerdanyola del Vallès (ES)
(72) Inventor: BOSCH TUBERT, Fàtima, E-08193 Cerdanyola del Vallès (ES); VILÀ PRATS, Laia, E-08193 Cerdanyola del Vallès (ES)
(86) International application number: PCT/EP2014/070531
(87) International publication number: WO 2015/044292

(56) References cited:
- WO-A2-2006/001982
- LI YU ET AL: "Hepatic overexpression of SIRT1 in mice attenuates endoplasmic reticulum stress and insulin resistance in the liver.", FASEB JOURNAL : OFFICIAL PUBLICATION OF THE FEDERATION OF AMERICAN SOCIETIES FOR EXPERIMENTAL BIOLOGY MAY 2011, vol. 25, no. 5, May 2011 (2011-05), pages 1664-1679, XP002733878, ISSN: 1530-6860
- YI LIU ET AL: "A one-step cloning method for the construction of somatic cell gene targeting vectors: application to production of human knockout cell lines", BMC BIOTECHNOLOGY, BIOMED CENTRAL LTD. LONDON, GB, vol. 12, no. 1, 9 October 2012 (2012-10-09), page 71, XP021121081, ISSN: 1472-6750, DOI: 10.1186/1472-6750-12-71
- Yoon et al: "Neuroprotective effects of molecular chaperones and anti-apoptotic genes BAG1, PINK1 and SIRT1 overexpression in rat models of Parkinson's Disease", 38th Annual Meeting of the Society-for-Neuroscience; Washington, DC, USA; November 15 -19, 2008 , vol. 38 18 November 2008 (2008-11-18), 2008, XP002733879, Retrieved from the Internet: URL:http://www.abstractsonline.com/plan/Vi ewAbstract.aspx?cKey=8c6aaf1a-6cd9-4573-b6 75-793bee0ed460&mID=1981&mKey=%7bAFEA068D- D012-4520-8E42-10E4D1AF7944%7d&sKey=1fcbf4 e5-b50c-44dc-9690-5f4e72576cdf [retrieved on 2014-12-17]
- VIL GBP A L ET AL: "237: AAV8-mediated Sirt1 overexpression in the liver prevents high-carbohydrate diet induced NAFLD", DIABETOLOGIA; 49TH ANNUAL MEEING OF THE EUROPEAN ASSOCIATION FOR THE STUDY OF DIABETES, SPRINGER, BERLIN, DE; BARCELONA, SPAIN, vol. 56, no. Suppl. 1, 27 September 2013 (2013-09-27), page S105, XP008173778, ISSN: 0012-186X, DOI: 10.1007/S00125-013-3012-Z
- VAN LINTHOUT S ET AL: "Persistent hepatic expression of human apo A-I after transfer with a helper-virus independent adenoviral vector.", GENE THERAPY NOV 2002, vol. 9, no. 22, November 2002 (2002-11), pages 1520-1528, ISSN: 0969-7128
- AL-DOSARI MOHAMMED ET AL: "Evaluation of viral and mammalian promoters for driving transgene expression in mouse liver.", BIOCHEMICAL AND BIOPHYSICAL RESEARCH COMMUNICATIONS 13 JAN 2006, vol. 339, no. 2, 13 January 2006 (2006-01-13), pages 673-678, ISSN: 0006-291X

### SEQUENCE LISTING

<110> Universitat Autònoma de Barcelona
<120> GENE THERAPY COMPOSITIONS FOR USE IN THE PREVENTION AND/OR TREATMENT OF
   NON-ALCOHOLIC FATTY LIVER DISEASE.
<130> EP-06637
<160> 55
<170> BiSSAP 1.2
<210> 1
   <211> 7908
   <212> DNA
   <213> Artificial Sequence
<220>
   <221> source
   <222> 1..7908
   <223> /mol_type="unassigned DNA" /note="Artificial plasmid pGG3-HCR/hAAT-OptSirt-1" /organism="Artificial Sequence"
<220>
   <221> promoter
   <222> 4321..5414
<220>
   <221> intron
   <222> 5458..5542
<220>
   <221> gene
   <222> 5683..7908
<220>
   <221> polyA_site
   <222> 31..197
<220>
   <221> misc_feature
   <222> 4116..4256
   <223> /standard_name="5'Inverted Terminal Repeat (ITR) AAV2 sequence"
<220>
   <221> misc_feature
   <222> 356..507
   <223> /standard_name="3'Inverted Terminal Repeat (ITR) AAV2 sequence"
<400> 1
   tcgagacgcg tgatatcgga tcccggccgg cggccgcttc cctttagtga gggttaatgc     60
   ttcgagcaga catgataaga tacattgatg agtttggaca aaccacaact agaatgcagt    120
   gaaaaaaatg ctttatttgt gaaatttgtg atgctattgc tttatttgta accattataa    180
   gctgcaataa acaagttaac aacaacaatt gcattcattt tatgtttcag gttcaggggg    240
   agatgtggga ggttttttaa agcaagtaaa acctctacaa atgtggtaaa atccgataag    300
   ggactagagc atggctacgt agataagtag catggcgggt taatcattaa ctacaaggaa    360
   cccctagtga tggagttggc cactccctct ctgcgcgctc gctcgctcac tgaggccggg    420
   cgaccaaagg tcgcccgacg cccgggcttt gcccgggcgg cctcagtgag cgagcgagcg    480
   cgccagctgg cgtaatagcg aagaggcccg caccgatcgc ccttcccaac agttgcgcag    540
   cctgaatggc gaatggaatt ccagacgatt gagcgtcaaa atgtaggtat ttccatgagc 600
   gtttttccgt tgcaatggct ggcggtaata ttgttctgga tattaccagc aaggccgata 660
   gtttgagttc ttctactcag gcaagtgatg ttattactaa tcaaagaagt attgcgacaa 720
   cggttaattt gcgtgatgga cagactcttt tactcggtgg cctcactgat tataaaaaca 780
   cttctcagga ttctggcgta ccgttcctgt ctaaaatccc tttaatcggc ctcctgttta 840
   gctcccgctc tgattctaac gaggaaagca cgttatacgt gctcgtcaaa gcaaccatag 900
   tacgcgccct gtagcggcgc attaagcgcg gcgggtgtgg tggttacgcg cagcgtgacc 960
   gctacacttg ccagcgccct agcgcccgct cctttcgctt tcttcccttc ctttctcgcc 1020
   acgttcgccg gctttccccg tcaagctcta aatcgggggc tccctttagg gttccgattt 1080
   agtgctttac ggcacctcga ccccaaaaaa cttgattagg gtgatggttc acgtagtggg 1140
   ccatcgccct gatagacggt ttttcgccct ttgacgttgg agtccacgtt ctttaatagt 1200
   ggactcttgt tccaaactgg aacaacactc aaccctatct cggtctattc ttttgattta 1260
   taagggattt tgccgatttc ggcctattgg ttaaaaaatg agctgattta acaaaaattt 1320
   aacgcgaatt ttaacaaaat attaacgtct acaatttaaa tatttgctta tacaatcttc 1380
   ctgtttttgg ggcttttctg attatcaacc ggggtacata tgattgacat gctagtttta 1440
   cgattaccgt tcatcgattc tcttgtttgc tccagactct caggcaatga cctgatagcc 1500
   tttgtagaga cctctcaaaa atagctaccc tctccggcat gaatttatca gctagaacgg 1560
   ttgaatatca tattgatggt gatttgactg tctccggcct ttctcacccg tttgaatctt 1620
   tacctacaca ttactcaggc attgcattta aaatatatga gggttctaaa aatttttatc 1680
   cttgcgttga aataaaggct tctcccgcaa aagtattaca gggtcataat gtttttggta 1740
   caaccgattt agctttatgc tctgaggctt tattgcttaa ttttgctaat tctttgcctt 1800
   gcctgtatga tttattggat gttggaatcg cctgatgcgg tattttctcc ttacgcatct 1860
   gtgcggtatt tcacaccgca tatggtgcac tctcagtaca atctgctctg atgccgcata 1920
   gttaagccag ccccgacacc cgccaacacc cgctgacgcg ccctgacggg cttgtctgct 1980
   cccggcatcc gcttacagac aagctgtgac cgtctccggg agctgcatgt gtcagaggtt 2040
   ttcaccgtca tcaccgaaac gcgcgagacg aaagggcctc gtgatacgcc tatttttata 2100
   ggttaatgtc atgataataa tggtttctta gacgtcaggt ggcacttttc ggggaaatgt 2160
   gcgcggaacc cctatttgtt tatttttcta aatacattca aatatgtatc cgctcatgag 2220
   acaataaccc tgataaatgc ttcaataata ttgaaaaagg aagagtatga gtattcaaca 2280
   tttccgtgtc gcccttattc ccttttttgc ggcattttgc cttcctgttt ttgctcaccc 2340
   agaaacgctg gtgaaagtaa aagatgctga agatcagttg ggtgcacgag tgggttacat 2400
   cgaactggat ctcaacagcg gtaagatcct tgagagtttt cgccccgaag aacgttttcc 2460
   aatgatgagc acttttaaag ttctgctatg tggcgcggta ttatcccgta ttgacgccgg 2520
   gcaagagcaa ctcggtcgcc gcatacacta ttctcagaat gacttggttg agtactcacc 2580
   agtcacagaa aagcatctta cggatggcat gacagtaaga gaattatgca gtgctgccat 2640
   aaccatgagt gataacactg cggccaactt acttctgaca acgatcggag gaccgaagga 2700
   gctaaccgct tttttgcaca acatggggga tcatgtaact cgccttgatc gttgggaacc 2760
   ggagctgaat gaagccatac caaacgacga gcgtgacacc acgatgcctg tagcaatggc 2820
   aacaacgttg cgcaaactat taactggcga actacttact ctagcttccc ggcaacaatt 2880
   aatagactgg atggaggcgg ataaagttgc aggaccactt ctgcgctcgg cccttccggc 2940
   tggctggttt attgctgata aatctggagc cggtgagcgt gggtctcgcg gtatcattgc 3000
   agcactgggg ccagatggta agccctcccg tatcgtagtt atctacacga cggggagtca 3060
   ggcaactatg gatgaacgaa atagacagat cgctgagata ggtgcctcac tgattaagca 3120
   ttggtaactg tcagaccaag tttactcata tatactttag attgatttaa aacttcattt 3180
   ttaatttaaa aggatctagg tgaagatcct ttttgataat ctcatgacca aaatccctta 3240
   acgtgagttt tcgttccact gagcgtcaga ccccgtagaa aagatcaaag gatcttcttg 3300
   agatcctttt tttctgcgcg taatctgctg cttgcaaaca aaaaaaccac cgctaccagc 3360
   ggtggtttgt ttgccggatc aagagctacc aactcttttt ccgaaggtaa ctggcttcag 3420
   cagagcgcag ataccaaata ctgtccttct agtgtagccg tagttaggcc accacttcaa 3480
   gaactctgta gcaccgccta catacctcgc tctgctaatc ctgttaccag tggctgctgc 3540
   cagtggcgat aagtcgtgtc ttaccgggtt ggactcaaga cgatagttac cggataaggc 3600
   gcagcggtcg ggctgaacgg ggggttcgtg cacacagccc agcttggagc gaacgaccta 3660
   caccgaactg agatacctac agcgtgagct atgagaaagc gccacgcttc ccgaagggag 3720
   aaaggcggac aggtatccgg taagcggcag ggtcggaaca ggagagcgca cgagggagct 3780
   tccaggggga aacgcctggt atctttatag tcctgtcggg tttcgccacc tctgacttga 3840
   gcgtcgattt ttgtgatgct cgtcaggggg gcggagccta tggaaaaacg ccagcaacgc 3900
   ggccttttta cggttcctgg ccttttgctg gccttttgct cacatgttct ttcctgcgtt 3960
   atcccctgat tctgtggata accgtattac cgcctttgag tgagctgata ccgctcgccg 4020
   cagccgaacg accgagcgca gcgagtcagt gagcgaggaa gcggaagagc gcccaatacg 4080
   caaaccgcct ctccccgcgc gttggccgat tcattaatgc agcagctgcg cgctcgctcg 4140
   ctcactgagg ccgcccgggc aaagcccggg cgtcgggcga cctttggtcg cccggcctca 4200
   gtgagcgagc gagcgcgcag agagggagtg gccaactcca tcactagggg ttccttgtag 4260
   ttaatgatta acccgccatg ctacttatct acgtagccat gctctagaca tggctcgaca 4320
   gatctgatat catcgatgaa ttcgagctcg gtacccggcc gcagatttag gtgacactat 4380
   agaatatgca tcactagtaa gcttgcgaat tccagtctac agagaggtct ctgacctctg 4440
   ccccagctcc aaggtcagca ggcagggagg gctgtgtgtt tgctgtttgc tgcttgcaat 4500
   gtttgcccat tttagggaca tgagtaggct gaagtttgtt cagtgtggac ttcagaggca 4560
   gcacacaaac agcaagcttg cgaattccag tctacagaga ggtctctgac ctctgcccca 4620
   gctccaaggt cagcaggcag ggagggctgt gtgtttgctg tttgctgctt gcaatgtttg 4680
   cccattttag ggacatgagt aggctgaagt ttgttcagtg tggacttcag aggcagcaca 4740
   caaacagcaa gcttgcgaat tccagtctac agagaggtct ctgacctctg ccccagctcc 4800
   aaggtcagca ggcagggagg gctgtgtgtt tgctgtttgc tgcttgcaat gtttgcccat 4860
   tttagggaca tgagtaggct gaagtttgtt cagtgtggac ttcagaggca gcacacaaac 4920
   agcaagcttt gctctagact ggaattcgtc gacgagctcc ctatagtgag tcgtattaga 4980
   ggccgactga cccggtaccc ggggatcttg ctaccagtgg aacagccact aaggattctg 5040
   cagtgagagc agagggccag ctaagtggta ctctcccaga gactgtctga ctcacgccac 5100
   cccctccacc ttggacacag gacgctgtgg tttctgagcc aggtacaatg actcctttcg 5160
   gtaagtgcag tggaagctgt acactgccca ggcaaagcgt ccgggcagcg taggcgggcg 5220
   actcagatcc cagccagtgg acttagcccc tgtttgctcc tccgataact ggggtgacct 5280
   tggttaatat tcaccagcag cctcccccgt tgcccctctg gatccactgc ttaaatacgg 5340
   acgaggacag ggccctgtct cctcagcttc aggcaccacc actgacctgg gacagtgaat 5400
   gtccccctga tctgcggccg tgactctctt aaggtagcct tgcagaagtt ggtcgtgagg 5460
   cactgggcag gtaagtatca aggttacaag acaggtttaa ggagaccaat agaaactggg 5520
   cttgtcgaga cagagaagac tcttgcgttt ctgataggca cctattggtc ttactgacat 5580
   ccactttgcc tttctctcca caggtgtcca ctcccagttc aattacagct cttaaggcta 5640
   gagtacttaa tacgactcac tataggctag cctcgacctc gaggggccac catggccgat 5700
   gaagtggctc tggcactgca ggccgctggc agtccttctg ccgcagctgc catggaagcc 5760
   gcctctcagc ctgccgacga gcccctgaga aagaggccca gaagggacgg ccctggcctg 5820
   ggaagaagcc ctggcgagcc ttcagctgcc gtggctcctg ccgctgccgg ctgtgaagcc 5880
   gcttctgctg ctgcccctgc cgctctgtgg cgagaagctg ctggcgccgc tgcctctgcc 5940
   gagagagagg ctcctgctac agccgtggcc ggagacggcg ataacggcag cggcctgaga 6000
   agagagccta gagccgccga cgacttcgac gacgacgagg gcgaggaaga ggacgaggcc 6060
   gctgctgccg cagcagctgc cgccatcggc tacagagaca acctgctgct gaccgacggc 6120
   ctgctgacaa acggcttcca cagctgcgag agcgacgacg atgacagaac cagccacgcc 6180
   tctagcagcg actggacccc cagacccaga atcggcccct acaccttcgt gcagcagcac 6240
   ctgatgatcg gcaccgaccc cagaaccatc ctgaaggacc tgctgcccga gacaatcccc 6300
   ccacccgagc tggacgacat gaccctgtgg cagatcgtga tcaacatcct gagcgagccc 6360
   cccaagagaa agaagcgcaa ggacatcaac accatcgagg acgccgtgaa gctgctgcag 6420
   gaatgcaaga aaatcatcgt gctgaccggc gctggcgtgt ccgtgtcttg cggcatcccc 6480
   gacttcagaa gcagggacgg catctacgcc agactggccg tggacttccc cgacctgccc 6540
   gatcctcagg ccatgttcga catcgagtac ttcagaaagg accccaggcc attcttcaag 6600
   ttcgccaaag agatctaccc cggccagttc cagcccagcc tgtgccacaa gtttatcgcc 6660
   ctgagcgaca aagagggcaa gctgctgaga aactacaccc agaacatcga caccctggaa 6720
   caggtggccg gcatccagag aatcctgcag tgtcacggca gcttcgccac cgccagctgc 6780
   ctgatctgca agtacaaggt ggactgcgag gccgtgcggg gcgacatctt caaccaggtg 6840
   gtgcccagat gccccaggtg ccctgccgat gagcctctgg ccatcatgaa gcccgagatc 6900
   gtgttcttcg gcgagaacct gcccgagcag ttccacagag ccatgaagta cgacaaggac 6960
   gaggtggacc tgctgatcgt gatcggcagc agcctgaaag tgcggcccgt ggccctgatc 7020
   cccagctcta tccctcacga ggtgccccag atcctgatca atagagagcc cctgccccat 7080
   ctgcacttcg acgtcgagct gctgggcgac tgcgacgtga tcatcaacga gctgtgccac 7140
   agactgggcg gcgagtacgc caagctgtgc tgcaaccctg tgaagctgag cgagatcacc 7200
   gagaagcccc ccaggcccca gaaagaactg gtgcacctgt ccgagctgcc ccccacccct 7260
   ctgcacatca gcgaggacag cagcagcccc gagagaaccg tgccccagga cagctccgtg 7320
   atcgccacac tggtggacca ggccaccaac aacaacgtga acgacctgga agtgtccgag 7380
   agcagctgcg tggaagagaa gccccaggaa gtgcagacca gcagaaacgt ggaaaacatc 7440
   aacgtcgaga accctgactt caaggccgtg ggcagctcca ccgccgacaa gaacgagaga 7500
   accagcgtgg ccgagacagt gcggaagtgc tggcccaaca gactggccaa agagcagatc 7560
   agcaagagac tggaaggcaa ccagtacctg ttcgtgcccc ccaaccggta catcttccac 7620
   ggcgccgagg tgtacagcga cagcgaggac gacgtgctga gcagctccag ctgcggcagc 7680
   aactccgaca gcggcacctg tcagagcccc agcctggaag aacctctgga agatgagagc 7740
   gagatcgagg aattctacaa cggcctcgag gacgacaccg agaggcctga gtgtgctggc 7800
   ggctctggct ttggcgccga cggcggagat caggaagtgg tcaacgaggc tatcgccacc 7860
   agacaggaac tgaccgacgt caactacccc agcgacaaga gctgaccc 7908
<210> 2
   <211> 20
   <212> DNA
   <213> Artificial Sequence
<220>
   <221> source
   <222> 1..20
   <223> /mol_type="unassigned DNA" /note="36b4 Forward primer" /organism="Artificial Sequence"
<400> 2
   tcccaccttg tctccagtct 20
<210> 3
   <211> 21
   <212> DNA
   <213> Artificial Sequence
<220>
   <221> source
   <222> 1..21
   <223> /mol_type="unassigned DNA" /note="36b4 Reverse Primer" /organism="Artificial Sequence"
<400> 3
   actggtctag gacccgagaa g 21
<210> 4
   <211> 19
   <212> DNA
   <213> Artificial Sequence
<220>
   <221> source
   <222> 1..19
   <223> /mol_type="unassigned DNA" /note="Acadl Forward Primer" /organism="Artificial Sequence"
<400> 4
   cccatggcat tagcctctt 19
<210> 5
   <211> 24
   <212> DNA
   <213> Artificial Sequence
<220>
   <221> source
   <222> 1..24
   <223> /mol_type="unassigned DNA" /note="Acadl Reverse Primer" /organism="Artificial Sequence"
<400> 5
   agaatagttc tgctgtgtcc tgag 24
<210> 6
   <211> 22
   <212> DNA
   <213> Artificial Sequence
<220>
   <221> source
   <222> 1..22
   <223> /mol_type="unassigned DNA" /note="Acadm Forward Primer" /organism="Artificial Sequence"
<400> 6
   agtaccctgt ggagaagctg at 22
<210> 7
   <211> 21
   <212> DNA
   <213> Artificial Sequence
<220>
   <221> source
   <222> 1..21
   <223> /mol_type="unassigned DNA" /note="Acadm Reverse Primer" /organism="Artificial Sequence"
<400> 7
   tcaatgtgct cacgagctat g 21
<210> 8
   <211> 19
   <212> DNA
   <213> Artificial Sequence
<220>
   <221> source
   <222> 1..19
   <223> /mol_type="unassigned DNA" /note="Acadvl Forward Primer" /organism="Artificial Sequence"
<400> 8
   ggtggtttgg gcctctcta 19
<210> 9
   <211> 20
   <212> DNA
   <213> Artificial Sequence
<220>
   <221> source
   <222> 1..20
   <223> /mol_type="unassigned DNA" /note="Acadvl Reverse Primer" /organism="Artificial Sequence"
<400> 9
   gggtaacgct aacaccaagg 20
<210> 10
   <211> 21
   <212> DNA
   <213> Artificial Sequence
<220>
   <221> source
   <222> 1..21
   <223> /mol_type="unassigned DNA" /note="Adipoq Forward Primer" /organism="Artificial Sequence"
<400> 10
   tgttcctctt aatcctgccc a 21
<210> 11
   <211> 21
   <212> DNA
   <213> Artificial Sequence
<220>
   <221> source
   <222> 1..21
   <223> /mol_type="unassigned DNA" /note="Adipoq Reverse Primer" /organism="Artificial Sequence"
<400> 11
   ccaacctgca caagttccct t 21
<210> 12
   <211> 20
   <212> DNA
   <213> Artificial Sequence
<220>
   <221> source
   <222> 1..20
   <223> /mol_type="unassigned DNA" /note="Ccl2 Forward Primer" /organism="Artificial Sequence"
<400> 12
   cccaatgagt aggctggaga 20
<210> 13
   <211> 20
   <212> DNA
   <213> Artificial Sequence
<220>
   <221> source
   <222> 1..20
   <223> /mol_type="unassigned DNA" /note="Ccl2 Reverse Primer" /organism="Artificial Sequence"
<400> 13
   tctggaccca ttccttcttg 20
<210> 14
   <211> 22
   <212> DNA
   <213> Artificial Sequence
<220>
   <221> source
   <222> 1..22
   <223> /mol_type="unassigned DNA" /note="Ccl5 Forward Primer" /organism="Artificial Sequence"
<400> 14
   cctcaccatc atcctcactg ca 22
<210> 15
   <211> 22
   <212> DNA
   <213> Artificial Sequence
<220>
   <221> source
   <222> 1..22
   <223> /mol_type="unassigned DNA" /note="Ccl5 Reverse Primer" /organism="Artificial Sequence"
<400> 15
   tcttctctgg gttggcacac ac 22
<210> 16
   <211> 20
   <212> DNA
   <213> Artificial Sequence
<220>
   <221> source
   <222> 1..20
   <223> /mol_type="unassigned DNA" /note="Cd68 Forward Primer" /organism="Artificial Sequence"
<400> 16
   ggggctcttg ggaactacac 20
<210> 17
   <211> 20
   <212> DNA
   <213> Artificial Sequence
<220>
   <221> source
   <222> 1..20
   <223> /mol_type="unassigned DNA" /note="Cd68 Reverse Primer" /organism="Artificial Sequence"
<400> 17
   caagccctct ttaagcccca 20
<210> 18
   <211> 18
   <212> DNA
   <213> Artificial Sequence
<220>
   <221> source
   <222> 1..18
   <223> /mol_type="unassigned DNA" /note="Cpt2 Forward Primer" /organism="Artificial Sequence"
<400> 18
   ccaaagaagc agcgatgg 18
<210> 19
   <211> 19
   <212> DNA
   <213> Artificial Sequence
<220>
   <221> source
   <222> 1..19
   <223> /mol_type="unassigned DNA" /note="Cpt2 Reverse Primer" /organism="Artificial Sequence"
<400> 19
   tagagctcag gcagggtga 19
<210> 20
   <211> 22
   <212> DNA
   <213> Artificial Sequence
<220>
   <221> source
   <222> 1..22
   <223> /mol_type="unassigned DNA" /note="F4/80 Forward Primer" /organism="Artificial Sequence"
<400> 20
   ctttggctat gggcttccag tc 22
<210> 21
   <211> 21
   <212> DNA
   <213> Artificial Sequence
<220>
   <221> source
   <222> 1..21
   <223> /mol_type="unassigned DNA" /note="F4/80 Reverse Primer" /organism="Artificial Sequence"
<400> 21
   gcaaggagga cagagtttat c 21
<210> 22
   <211> 24
   <212> DNA
   <213> Artificial Sequence
<220>
   <221> source
   <222> 1..24
   <223> /mol_type="unassigned DNA" /note="Hifla Forward Primer" /organism="Artificial Sequence"
<400> 22
   gcactagaca aagttcacct gaga 24
<210> 23
   <211> 20
   <212> DNA
   <213> Artificial Sequence
<220>
   <221> source
   <222> 1..20
   <223> /mol_type="unassigned DNA" /note="Hif1a-Reverse Primer" /organism="Artificial Sequence"
<400> 23
   cgctatccac atcaaagcaa 20
<210> 24
   <211> 21
   <212> DNA
   <213> Artificial Sequence
<220>
   <221> source
   <222> 1..21
   <223> /mol_type="unassigned DNA" /note="Il1b Forward Primer" /organism="Artificial Sequence"
<400> 24
   tgtaatgaaa gacggcacac c 21
<210> 25
   <211> 21
   <212> DNA
   <213> Artificial Sequence
<220>
   <221> source
   <222> 1..21
   <223> /mol_type="unassigned DNA" /note="Il1b Reverse Primer" /organism="Artificial Sequence"
<400> 25
   tcttctttgg gtattgcttg g 21
<210> 26
   <211> 21
   <212> DNA
   <213> Artificial Sequence
<220>
   <221> source
   <222> 1..21
   <223> /mol_type="unassigned DNA" /note="Il10 Forward Primer" /organism="Artificial Sequence"
<400> 26
   ctatgctgcc tgctcttact g 21
<210> 27
   <211> 22
   <212> DNA
   <213> Artificial Sequence
<220>
   <221> source
   <222> 1..22
   <223> /mol_type="unassigned DNA" /note="Il10 Reverse Primer" /organism="Artificial Sequence"
<400> 27
   aacccaagta acccttaaag tc 22
<210> 28
   <211> 19
   <212> DNA
   <213> Artificial Sequence
<220>
   <221> source
   <222> 1..19
   <223> /mol_type="unassigned DNA" /note="Lep Forward Primer" /organism="Artificial Sequence"
<400> 28
   gagacccctg tgtcggttc 19
<210> 29
   <211> 22
   <212> DNA
   <213> Artificial Sequence
<220>
   <221> source
   <222> 1..22
   <223> /mol_type="unassigned DNA" /note="Lep Reverse Primer" /organism="Artificial Sequence"
<400> 29
   ctgcgtgtgt gaaatgtcat tg 22
<210> 30
   <211> 20
   <212> DNA
   <213> Artificial Sequence
<220>
   <221> source
   <222> 1..20
   <223> /mol_type="unassigned DNA" /note="mCpt1 Forward Primer" /organism="Artificial Sequence"
<400> 30
   gcacaccagg cagtagcttt 20
<210> 31
   <211> 23
   <212> DNA
   <213> Artificial Sequence
<220>
   <221> source
   <222> 1..23
   <223> /mol_type="unassigned DNA" /note="mCpt1 Reverse Primer" /organism="Artificial Sequence"
<400> 31
   caggagttga ttccagacag gta 23
<210> 32
   <211> 18
   <212> DNA
   <213> Artificial Sequence
<220>
   <221> source
   <222> 1..18
   <223> /mol_type="unassigned DNA" /note="Nos2 Forward Primer" /organism="Artificial Sequence"
<400> 32
   ctttgccacg gacgagac 18
<210> 33
   <211> 22
   <212> DNA
   <213> Artificial Sequence
<220>
   <221> source
   <222> 1..22
   <223> /mol_type="unassigned DNA" /note="Nos2 Reverse Primer" /organism="Artificial Sequence"
<400> 33
   tcattgtact ctgagggctg ac 22
<210> 34
   <211> 20
   <212> DNA
   <213> Artificial Sequence
<220>
   <221> source
   <222> 1..20
   <223> /mol_type="unassigned DNA" /note="Nrf1 Forward Primer" /organism="Artificial Sequence"
<400> 34
   ctgcaggtcc tgtgggaatg 20
<210> 35
   <211> 20
   <212> DNA
   <213> Artificial Sequence
<220>
   <221> source
   <222> 1..20
   <223> /mol_type="unassigned DNA" /note="Nrf1 Reverse Primer" /organism="Artificial Sequence"
<400> 35
   actcgcgtcg tgtactcatc 20
<210> 36
   <211> 20
   <212> DNA
   <213> Artificial Sequence
<220>
   <221> source
   <222> 1..20
   <223> /mol_type="unassigned DNA" /note="Pdk4 Forward Primer" /organism="Artificial Sequence"
<400> 36
   ccttcaccac atgctcttcg 20
<210> 37
   <211> 20
   <212> DNA
   <213> Artificial Sequence
<220>
   <221> source
   <222> 1..20
   <223> /mol_type="unassigned DNA" /note="Pdk4 Reverse Primer" /organism="Artificial Sequence"
<400> 37
   cggttttctt gatgctcgac 20
<210> 38
   <211> 22
   <212> DNA
   <213> Artificial Sequence
<220>
   <221> source
   <222> 1..22
   <223> /mol_type="unassigned DNA" /note="Ppargcla Forward Primer" /organism="Artificial Sequence"
<400> 38
   ataccgcaaa gagcacgaga ag 22
<210> 39
   <211> 25
   <212> DNA
   <213> Artificial Sequence
<220>
   <221> source
   <222> 1..25
   <223> /mol_type="unassigned DNA" /note="Ppargcla Reverse Primer" /organism="Artificial Sequence"
<400> 39
   ctcaagagca gcgaaagcgt cacag 25
<210> 40
   <211> 19
   <212> DNA
   <213> Artificial Sequence
<220>
   <221> source
   <222> 1..19
   <223> /mol_type="unassigned DNA" /note="Ppara Forward Primer" /organism="Artificial Sequence"
<400> 40
   tcggcgaact attcggctg 19
<210> 41
   <211> 20
   <212> DNA
   <213> Artificial Sequence
<220>
   <221> source
   <222> 1..20
   <223> /mol_type="unassigned DNA" /note="Ppara Reverse Primer" /organism="Artificial Sequence"
<400> 41
   gcacttgtga aaacggcagt 20
<210> 42
   <211> 21
   <212> DNA
   <213> Artificial Sequence
<220>
   <221> source
   <222> 1..21
   <223> /mol_type="unassigned DNA" /note="Ppard Forward Primer" /organism="Artificial Sequence"
<400> 42
   tccatcgtca acaaagacgg g 21
<210> 43
   <211> 21
   <212> DNA
   <213> Artificial Sequence
<220>
   <221> source
   <222> 1..21
   <223> /mol_type="unassigned DNA" /note="Ppard Reverse Primer" /organism="Artificial Sequence"
<400> 43
   acttgggctc aatgatgtca c 21
<210> 44
   <211> 18
   <212> DNA
   <213> Artificial Sequence
<220>
   <221> source
   <222> 1..18
   <223> /mol_type="unassigned DNA" /note="Sirt3 Forward Primer" /organism="Artificial Sequence"
<400> 44
   tcctctgaaa ccggatgg 18
<210> 45
   <211> 20
   <212> DNA
   <213> Artificial Sequence
<220>
   <221> source
   <222> 1..20
   <223> /mol_type="unassigned DNA" /note="Sirt3 Reverse Primer" /organism="Artificial Sequence"
<400> 45
   tcccacacag agggatatgg 20
<210> 46
   <211> 19
   <212> DNA
   <213> Artificial Sequence
<220>
   <221> source
   <222> 1..19
   <223> /mol_type="unassigned DNA" /note="Sirt4 Forward Primer" /organism="Artificial Sequence"
<400> 46
   tgatgtccaa aggctggaa 19
<210> 47
   <211> 18
   <212> DNA
   <213> Artificial Sequence
<220>
   <221> source
   <222> 1..18
   <223> /mol_type="unassigned DNA" /note="Sirt4 Reverse Primer" /organism="Artificial Sequence"
<400> 47
   agagttggag cggcattg 18
<210> 48
   <211> 19
   <212> DNA
   <213> Artificial Sequence
<220>
   <221> source
   <222> 1..19
   <223> /mol_type="unassigned DNA" /note="Sirt6 Forward Primer" /organism="Artificial Sequence"
<400> 48
   gacctgatgc tcgctgatg 19
<210> 49
   <211> 20
   <212> DNA
   <213> Artificial Sequence
<220>
   <221> source
   <222> 1..20
   <223> /mol_type="unassigned DNA" /note="Sirt6 Reverse Primer" /organism="Artificial Sequence"
<400> 49
   ggtacccagg gtgacagaca 20
<210> 50
   <211> 25
   <212> DNA
   <213> Artificial Sequence
<220>
   <221> source
   <222> 1..25
   <223> /mol_type="unassigned DNA" /note="Tnfa Forward Primer" /organism="Artificial Sequence"
<400> 50
   catcttctca aaattcgagt gacaa 25
<210> 51
   <211> 23
   <212> DNA
   <213> Artificial Sequence
<220>
   <221> source
   <222> 1..23
   <223> /mol_type="unassigned DNA" /note="Tnfa Reverse Primer" /organism="Artificial Sequence"
<400> 51
   tgggagtaga caaggtacaa ccc 23
<210> 52
   <211> 20
   <212> DNA
   <213> Artificial Sequence
<220>
   <221> source
   <222> 1..20
   <223> /mol_type="unassigned DNA" /note="Ucp2 Forward Primer" /organism="Artificial Sequence"
<400> 52
   actttccctc tggataccgc 20
<210> 53
   <211> 20
   <212> DNA
   <213> Artificial Sequence
<220>
   <221> source
   <222> 1..20
   <223> /mol_type="unassigned DNA" /note="Ucp2 Reverse Primer" /organism="Artificial Sequence"
<400> 53
   acggaggcaa agctcatctg 20
<210> 54
   <211> 20
   <212> DNA
   <213> Artificial Sequence
<220>
   <221> source
   <222> 1..20
   <223> /mol_type="unassigned DNA" /note="Ucp3 Forward Primer" /organism="Artificial Sequence"
<400> 54
   ctgcaccgcc agatgagttt 20
<210> 55
   <211> 21
   <212> DNA
   <213> Artificial Sequence
<220>
   <221> source
   <222> 1..21
   <223> /mo1_type="unassigned DNA" /note="Ucp3 Reverse Primer" /organism="Artificial Sequence"
<400> 55
   atcatggctt gaaatcggac c 21

## Description

### FIELD OF THE INVENTION

The present invention pertains to the field of prevention and/or treatment of diseases, and particularly the treatment of non-alcoholic fatty liver disease (NAFLD) by gene therapy compositions. More specifically, the invention pertains to gene therapy compositions that comprise vectors, specifically viral vectors, such as parvoviral vectors, containing the gene encoding Sirtuin 1 (Sirt1) in the prevention and/or treatment of NAFLD.

### STATE OF THE ART

Non-alcoholic fatty liver disease (NAFLD), characterized by an aberrant lipid storage in hepatocytes, is currently the most common liver disease worldwide with a prevalence increasing in an alarming rate. Variations in dietary habits such as the increased consumption of carbohydrates, mainly in form of fructose or sucrose intake, together with a sedentary lifestyle, are in part responsible for the increased frequency of metabolic syndrome and its hepatic component, NAFLD. Furthermore, NAFLD can progress to NASH (Non Alcoholic Steatohepatitis) which is characterized by the appearance of necroinflammation in the liver. This inflammation, together with the accumulation of lipid species in the liver, could trigger the onset of hepatic insulin resistance and type 2 diabetes (Lim JS. et al. Nat Rev Gastroenterol Hepatol;2010; 7:251-264;).

Unfortunately, till date, the only treatment able to reduce or reverse liver steatosis is based on increasing physical activity and changing nutrient consumption to gradually reduce body weight and hepatic lipid accumulation (Deng XQ,. et al. Liver Int 2007;27:708-715; Zivkovic AM, et al. Am J Clin Nutr 2007;86:285-300; Zelber-Sagi S, et al. World J Gastroenterol;17:3377-3389). The reduction of body weight may be achieved by caloric restriction (CR). However, the exact effects of CR and the underlying mechanisms are largely unknown. One of the key genes involved in numerous effects of CR is Sirt1, a homologue of the yeast silent information regulator 2 (Cohen HY, et al. Science 2004;305:390-392; Nogueiras R, er al. Physiol Rev;2012;92:1479-1514). Sirt1 is a member of Sirtuins, a family of protein deacetylases and ADP-ribosyltransferases, which are involved in different pathways such as cellular metabolism, stress response, lifespan and caloric restriction (Yamamoto H, et al. Mol Endocrinol 2007;21:1745-1755;

Lomb DJ, et al. Biochim Biophys Acta;2010;1804:1652-1657). In mammals, seven sirtuins (Sirt1-7) have been identified. Sirt1 possesses a NAD+-dependent deacetylase activity and is able to modulate gene expression in several key metabolic tissues such as liver, skeletal muscle and white adipose tissue (WAT) by deacetylating histones, proteins and transcription factors. According to the energetic state of the cell, Sirt1 may act on the nuclear factor-κβ (NF-κβ) and the peroxisome proliferative activated receptor, gamma, coactivator 1 alpha (PPARGC1A).

To date, in the state of the art, there are many studies that try to elucidate the role of Sirt1 using transgenic mice as a tool and these studies are divided according to the Sirt1 gain or loss of function used strategy.

In this sense, Xu et al. (Xu et al. Endocrinology; 2010;151(6):2504-14), showed that Sirt1 heterozygous mice (SIRT1+/-) showed an increase in body fat content probably due to the reduction detected in energy expenditure. These mice showed severe hepatic steatosis due to an increase in lipogenesis and liver inflammation when fed with a high fat diet. In the same way, Purushotham et al. (Purushotham et al. FASEB J. 2012;26(2): 656-67) obtained similar results in Sirt1 heterozygous mice (SIRT1+/-) showing that the systemic Sirt1 insufficiency resulted in the disruption of energy homeostasis leading to a more insulin resistant and obese mice when fed under a high fat diet. Thus, the reduction in whole body Sirt1 expression promotes the development of obesity and NAFLD, increases inflammation and reduces insulin sensitivity.

Other groups, instead of analysing the global role of Sirt1 (using Sirt1 heterozygous mice), have developed liver-specific Sirt1 knockout mice models to analyse the role of Sirt1 specifically in the liver. Purushotham et al. (Purushotham et al. Cell Metab. 2009; 9(4): 327-38), described that when challenged with a high-fat diet, liver-specific Sirt1 knockout mice develop hepatic steatosis, hepatic inflammation, and endoplasmic reticulum stress by altering PPARα and PGC1-α signaling. In the same way, Wang, R. et al (Wang, R. et al. J Clin Invest; 2011:121(11): 4477-90), disclosed that liver-specific Sirt1 knockout mice results in hyperglycemia, oxidative damage, and insulin resistance as a consequence of an increase in the gluconeogenic process. Therefore, hepatic Sirt1 deletion induces hepatic steatosis and the alteration of glucose homeostasis.

On the other hand, there are many studies that tried to elucidate the role of Sirt1 using transgenic mice which overexpress Sirt1 ubiquitously. Pfluger PT. et al (Pfluger PT. et al. Proc Natl Acad Sci USA;2008; 105(28): 9793-8), showed that Sirt1 transgenic mice were able to avoid the development of insulin resistance and NAFLD in high fat diet fed mice and db/db mice by down regulating the expression of lipogenic genes like SREBP-1c and ChREBP. Moreover, Sirt1 overexpression also reduced hepatic inflammation (NFκb activity) and stimulated lipid oxidation by increasing PGC-1a activity. Sirt1 gain of function also displayed reductions in blood cholesterol, adipokines, insulin and fasted glucose levels. Moreover, the animals became more glucose tolerant and insulin sensitive. Some of these effects were due to the increase in β-oxidation activity detected in peripheral tissues like liver and skeletal muscle (Bordone et al. Aging Cell;2007;6(6): 759-67; Banks et al. Cell Metab.2008; 8(4): 333-41.2008).

The prior art also includes Li et al, Faseb J, 25, 2011, 1664-1678 disclosing adenovirus mediated expression of SIRT1, and effects thereof in attenuating hepatic steatosis and insulin resistance.

Thus, an attractive approach to mimic caloric restriction may be the overexpression of Sirt1. Although, as we indicated previously, there are many studies demonstrating that whole-body activation of Sirt1 triggers beneficial effects on metabolism, tissue-specific actions of Sirt1 overexpression remain to be elucidated. The problem of the state of the art is to find an effective long-term Sirt1 overexpression, specifically in the liver, for use in the prevention and/or treatment of NAFLD and related diseases selected from insulin resistance, diabetes mellitus, metabolic syndrome and hyperlipidemia, induced by a high caloric diet. The solution proposed by the present disclosure is the use of parvoviral vectors, preferably, adeno-associated viral (AAV) vectors, containing the gene encoding Sirt1 specifically in the liver, operably linked to a liver-specific promoter, for use in the prevention and/or treatment of NAFLD.

### DESCRIPTION OF THE INVENTION

### BRIEF DESCRIPTION OF THE INVENTION

In a first aspect, the disclosure relates to a viral construct comprising a nucleotide sequence encoding Sirtuin 1 (Sirt1) or a functionally equivalent variant thereof, which is operably linked to a liver-specific promoter.

In a first embodiment, the invention relates to a viral construct comprising a nucleotide sequence encoding Sirtuin 1 (Sirt1) wherein the nucleotide sequence has at least 60% identity, preferably at least 70% identity, more preferably at least 85% identity, even more preferably at least 95% identity with the nucleotide sequence described in NCBI under accession number AF083106, wherein said nucleotide sequence is operably linked to an alphal-antitrypsin liver-specific promoter, and wherein said viral construct is an AAV. In this sense, specific overexpression of Sirt1 in the liver may improve NAFLD. In the present disclosure, the inventors took advantage of the use of a combination of parvoviral vector, preferably, adeno-associated viral vectors, allowing a long-term expression of the transgene and a liver-specific promoter, thus restricting Sirt1 overexpression to the liver. AAV vectors are recognized as the gene transfer vectors of choice since they have shown the best safety and efficacy profile for the delivery of genes *in vivo.* The combination of invention has a great advantage related to the use of other viral vectors, such as the adenoviral vectors. Indeed, adenoviral vectors only achieve short term expression due to its high immunogenicity, whereas AAV vectors mediate long-term transgene expression because of their lack of pathogenicity and low immunogenicity.

In a second embodiment, the invention relates to a virion comprising the viral construct, according to the present invention, packaged inside a capsid and, optionally a lipidic envelope surrounding said capsid.

In still another embodiment, the invention relates to the viral construct or the virion of the invention, for use as medicament. In further embodiments, the invention relates to a pharmaceutical composition comprising the viral construct or the virion as defined in the present invention and a pharmaceutically acceptable carrier.

The disclosure also deals with a method for the treatment and/or prevention of NAFLD and related diseases, selected from insulin resistance, diabetes mellitus, metabolic syndrome and hyperlipidemia, comprising the administration to a subject in need thereof of an effective dose of the viral construct or the virion, as defined in the present invention.

### DESCRIPTION OF THE FIGURES

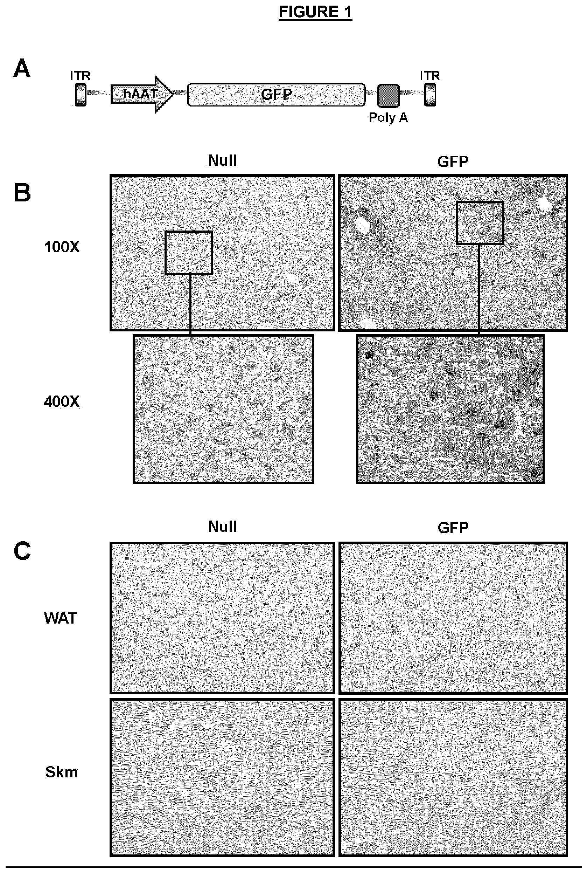

**FIGURE 1****. AAV8-hAAT-GFP injection achieves specific GFP expression in the livers of injected mice. (A)** Schematic view of the AAV8 vector used to overexpress green fluorescent protein (GFP) transgene under the control of the liver specific hAAT promoter. The AAV8 vectors AAV8-hAAT-null and AAV8-hAAT-GFP were injected at a dose of 5x10¹¹vg/mice. **(B-C)** Immunohistochemistry analysis for GFP (darker) was performed in **(B)** liver (representative images at original magnification x100 and x400), **(C)** epididymal WAT and skeletal muscle (representative images at original magnification x100) of mice injected with null or GFP vectors, respectively.

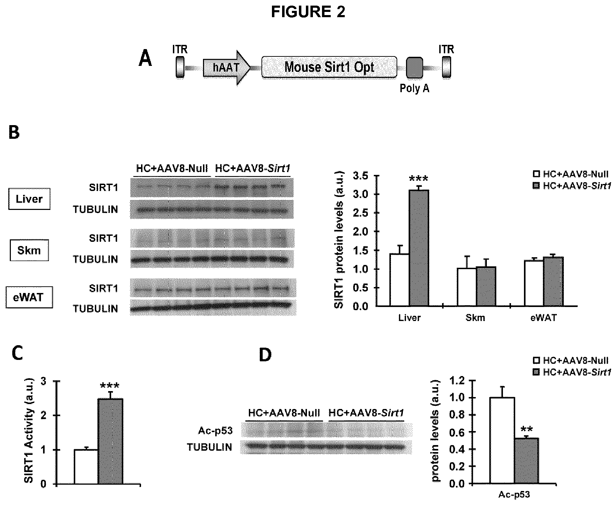

**FIGURE 2****. Systemic administration of AAV8-hAAT-*Sirt1* vectors led to specific SIRT1 overexpression in the liver. (A)** Schematic view of the AAV8 vector used to overexpress the optimized Sirt1 murine transgene under the control of the liver specific hAAT promoter. **(B)** A representative Western blot and its quantification are shown for SIRT1 in the liver, skeletal muscle (Skm) and epididymal white adipose tissue (eWAT) of mice fed with a high carbohydrate (HC) diet and treated with AAV8-hAAT-Null (AAV8-Null) or AAV8-hAAT-*Sirt1* vectors (AAV8-*Sirt1*) at a dose of 5x10¹¹ vg/mice for 15 weeks. **(C)** SIRT1 deacetylation activity in liver crude nuclear extracts. **(D)** Representative Western blot and quantification of Acetyl-p53-Lys³⁷⁹ protein levels in nuclear fractions from livers of AAV8-Null- and AAV8-*Sirt1*-treated mice, using tubulin as a loading control. a. u., arbitrary units. Data represent the mean ± SEM of at least four animals per group. ***p*<0.01 and ****p*<0.001 *vs.* CT group.

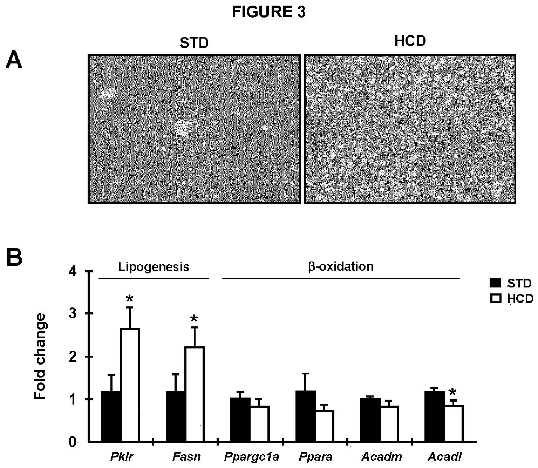

**FIGURE 3****. HCD induces hepatic lipid accumulation in mice. (A)** Representative sections of livers, stained with hematoxylin-eosin (original magnification x100) from standard (STD) and high carbohydrate diet (HCD) fed mice. **(B)** mRNA expression levels of *Lpk, Fasn, Ppargc1a, Ppara, Acadm* and *Acadl* were analyzed in livers of STD and HCD fed mice by quantitative real-time PCR and normalized by 36b4 expression. Data represent the mean±SEM of at least 4 animals per group. *p< 0.05 vs. STD group.

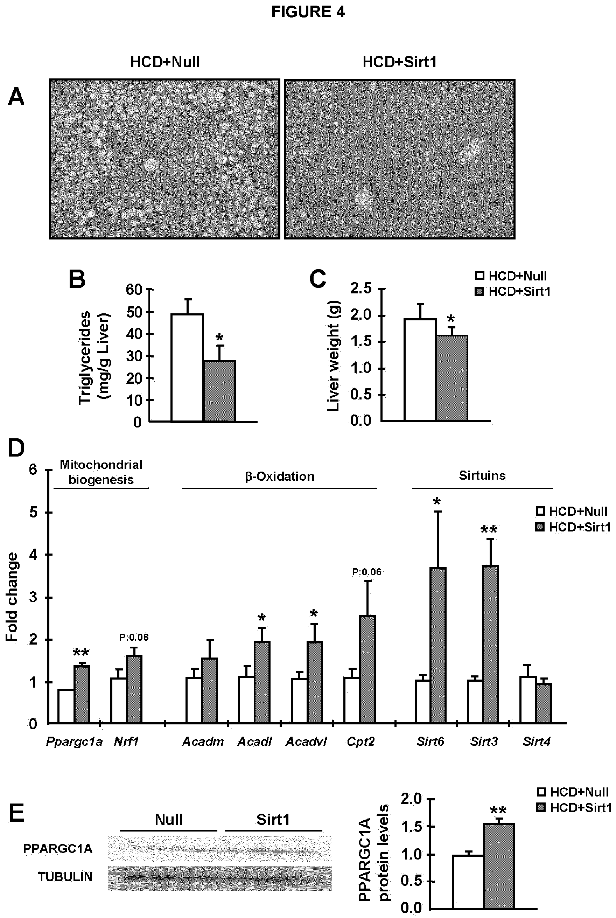

**FIGURE 4****. Sirt1 mice showed reduced hepatic lipid accumulation compared with Null mice. (A)** Representative sections of liver, stained with hematoxylin-eosin (original magnification x100) from Null and Sirt1 mice fed with a high carbohydrate diet (HCD). **(B)** Liver tissue weight. **(C)** Liver triglyceride content was determined as indicated in Example 1. **(D)** mRNA expression of *Ppargc1a, Nrf1, Acadm, Acadl, Acadvl, Cpt2, Sirt3, Sirt4* and *Sirt6* were analyzed in the liver of Null and Sirt1 mice by quantitative real-time PCR and normalized by 36b4 expression. **(E)** Representative western blot and quantification of hepatic PPARGC1a protein levels of Null and Sirt1 mice fed with a HCD, using tubulin as a loading control. Data represent the mean±SEM of at least 4 animals per group. *p< 0.05 and **p< 0.01 vs. CT group

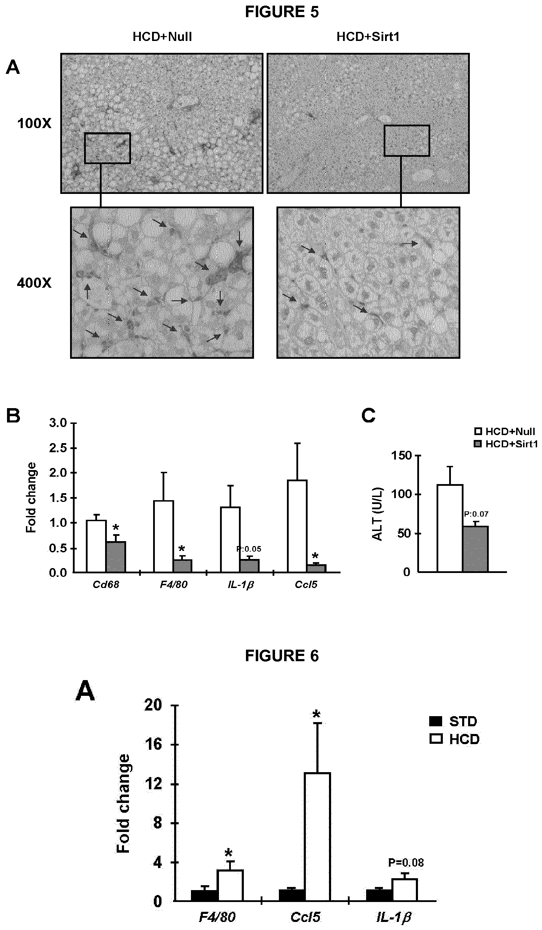

**FIGURE 5****. Lower macrophage infiltration and inflammation in livers of Sirt1 mice. (A)** MAC-2 immunohistochemistry was performed in livers of Null and Sirt1 mice fed under a high carbohydrate diet (HCD) (representative images at original magnification x100 and x400). Red arrows indicate MAC-2 signal. **(B)** *Cd68, F4*/*80, II1b* and *Ccl5* mRNA expression levels in liver of Null and Sirt1 mice were analyzed by quantitative real-time PCR and normalized by 36b4 expression. **(C)** Serum ALT levels of Null and Sirt1 mice were analyzed as indicated in Example 1. Data represent the mean±SEM of at least 4 animals per group. *p< 0.05 vs. CT group.
**FIGURE 6****. Hepatic inflammation is up-regulated by HCD. (A)** The expression levels of *F4*/*80, Ccl5* and *II1b* were analyzed in the livers of mice fed with a standard (STD) or a high carbohydrate diet (HCD) by quantitative real-time PCR and normalized by *36b4* expression. Data represent the mean±SEM of at least 4 animals per group. *p< 0.05 vs. STD group.

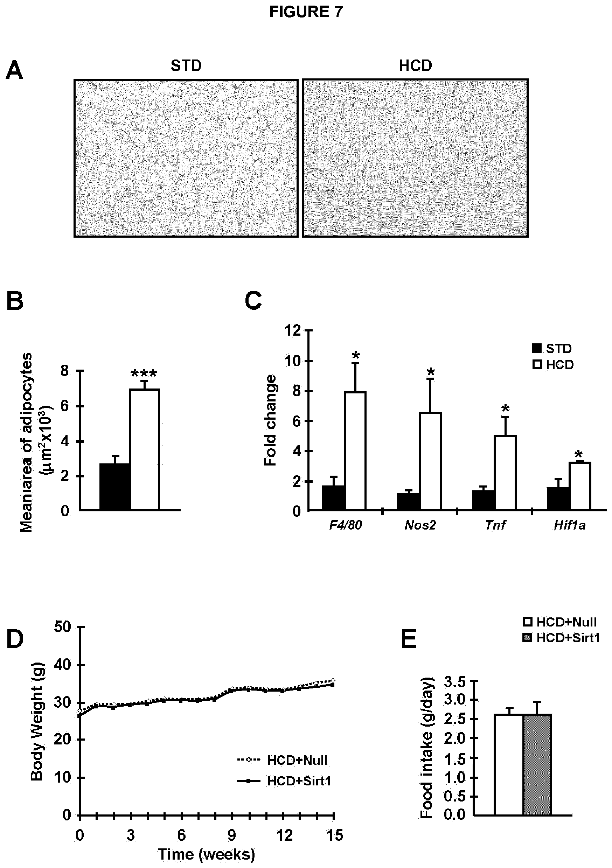

**FIGURE 7****. HCD induces WAT hypertrophy and inflammation. (A)** Representative sections of epididymal WAT, stained with hematoxylin-eosin (original magnification x100) from standard (STD) and (HCD) fed mice. **(B)** Mean adipocyte area, calculated as described in Example 1. **(C)** *F4*/*80, Nos2, Tnf* and *Hif1a* mRNA levels were checked in epididymal WAT from standard (STD) and (HCD) fed mice by quantitative real-time PCR and normalized by *36b4* expression. Data represent the mean±SEM of at least four animals per group. **(D-E)** Body weight gain **(D)** and food intake **(E)** of Null and Sirt1 mice fed under a high carbohydrate diet (HCD) for 15 weeks. Data represent the mean±SEM of at least 10 animals per group. *p< 0.05 vs. STD group; ***p< 0.001 vs. STD group.

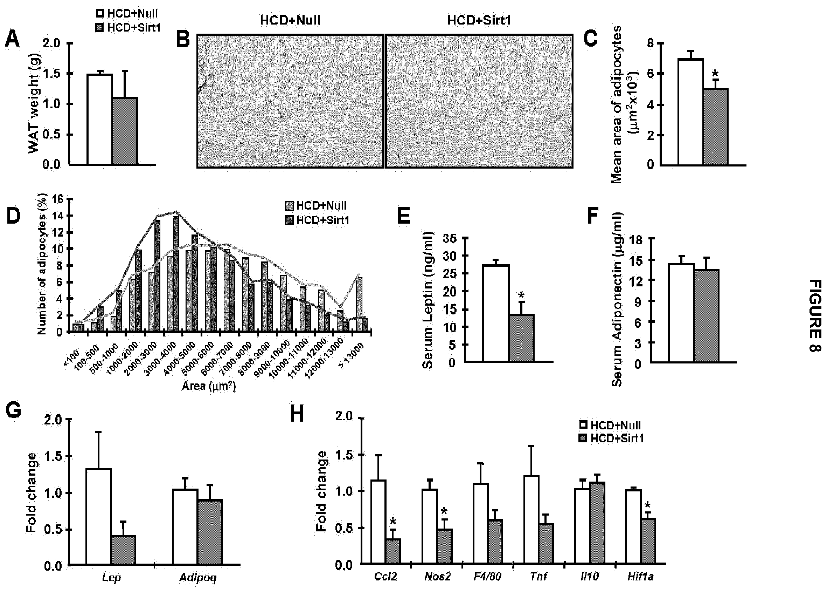

**FIGURE 8****. Reduced adipocyte size and inflammation in epididymal adipose tissue of Sirt1 mice. (A)** Epididymal WAT weight. **(B)** Representative sections of epididymal WAT, stained with hematoxylin-eosin (original magnification x100) from Null and Sirt1 mice fed with a high carbohydrate diet (HCD). (C) Mean adipocyte area, calculated as described in Example 1. **(D)** Frequency distribution of adipocyte area in Null and Sirt1 mice fed with a HCD was calculated as described in Example 1. **(E-F)** Serum leptin **(E)** and adiponectin **(F)** levels. **(G)** *Lep* and *Adipoq* mRNA expression in epididymal WAT of Null and Sirt1 mice were analyzed by quantitative real-time PCR and normalized by *36b4* expression. **(H)** *Ccl2, Nos2, F4*/*80, Tnfa, II10* and *Hif1a* mRNA levels were checked in epididymal WAT by quantitative real-time PCR and normalized by *36b4* expression. Data represent the mean±SEM of at least four animals per group. *p< 0.05 vs. CT group.

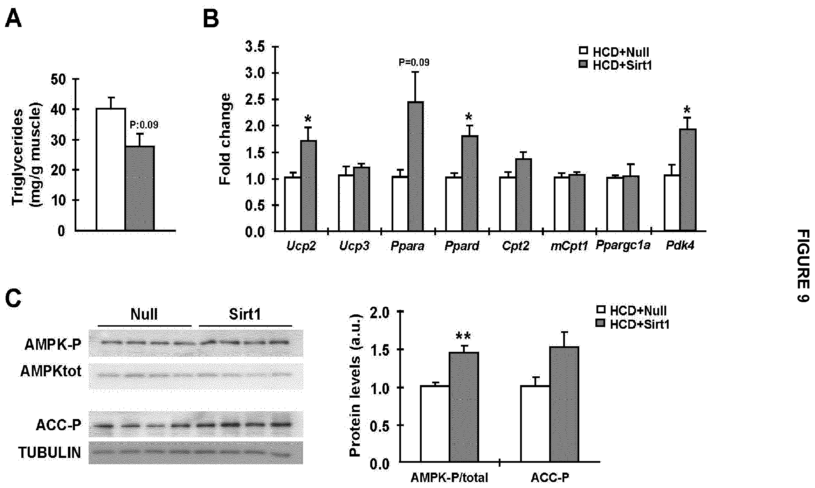

**FIGURE 9****. Sirt1 mice showed increased β-oxidation capacity in skeletal muscle. (A)** Triglyceride content in skeletal muscle was determined as described in Example 1. **(B)** Expression levels of *Ucp2, Ucp3, Ppara, Ppard, Cpt2, mCpt1, Ppargc1a* and *Pdk4* were analyzed in skeletal muscle of Null and Sirt1 mice by quantitative real-time PCR and normalized by 36b4 expression. **(C)** Representative western blots are shown for total AMPK (AMPKtot), phosphorylated AMPK (AMPK-P) and phosphorylated ACC (ACC-P) protein levels. On the right of the figure, a bar plot shows the ratio AMPK-P/total and the protein levels of ACC-P in skeletal muscle of Null and Sirt1 mice, using tubulin as a loading control. Data represent the mean±SEM of at least four animals per group. *p< 0.05 vs. CT group; **p< 0.01 vs. CT group.

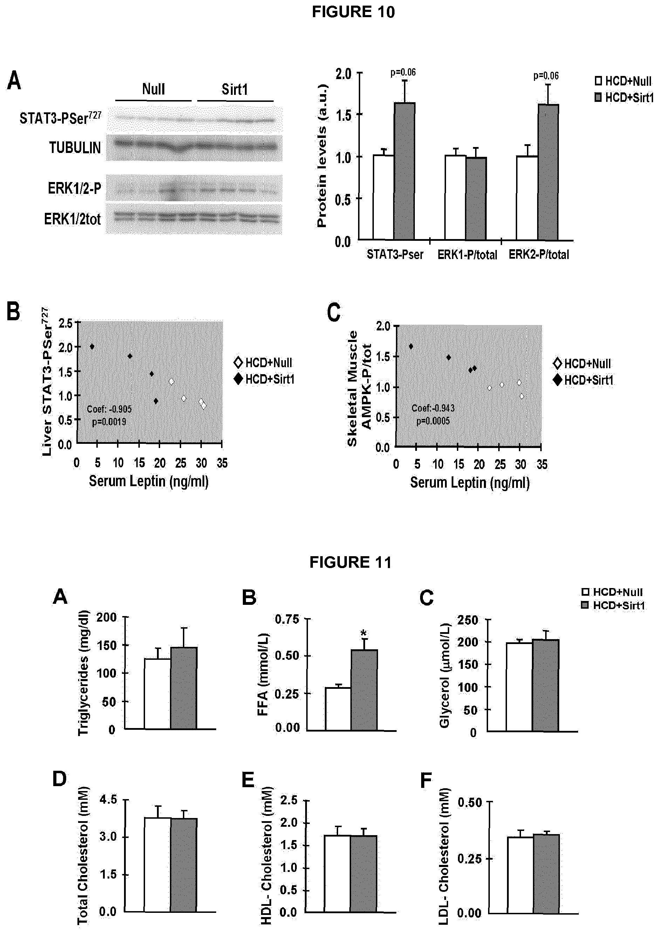

**FIGURE 10****. Hepatic Sirt1 overexpression counteracts the development of leptin resistance induced by HCD. (A)** Representative western blots are shown for the active and phosphorylated form of STAT3 at the Serine 727 residue (STAT3-PSer⁷²⁷), phosphorylated ERK1/2 (ERK1/2-P) and total ERK1/2 (ERK1/2tot) protein levels in liver of Null and Sirt1 mice fed with a high carbohydrate diet (HCD). On the right of the figure, a bar plot shows the protein levels of nuclear STAT3-PSer⁷²⁷ (normalized by tubulin protein levels) and the ratios ERK1-P/total and ERK2-P/total expressed as the mean±SEM of at least 4 animals per group. **(B)** Correlation analyses between serum leptin levels and hepatic protein levels of STAT3-PSer⁷²⁷ from Null and Sirt1 mice fed under a HCD. **(C)** Correlation analyses between serum leptin levels and the ratio AMPK-P/total in skeletal muscle from Null and Sirt1 mice fed under a HCD.
**FIGURE 11****. Levels of circulating metabolites were determined in Null and Sirt1 mice fed with a HCD. (A-F)** Serum levels of triglycerides **(A),** FFAs **(B),** glycerol **(C),** total cholesterol, **(D)** HDL cholesterol **(E)** and LDL-cholestrol **(F)** were analyzed as indicated in Example 1. Data represent the mean±SEM of at least 4 animals per group. *p<0.05 vs. CT group.

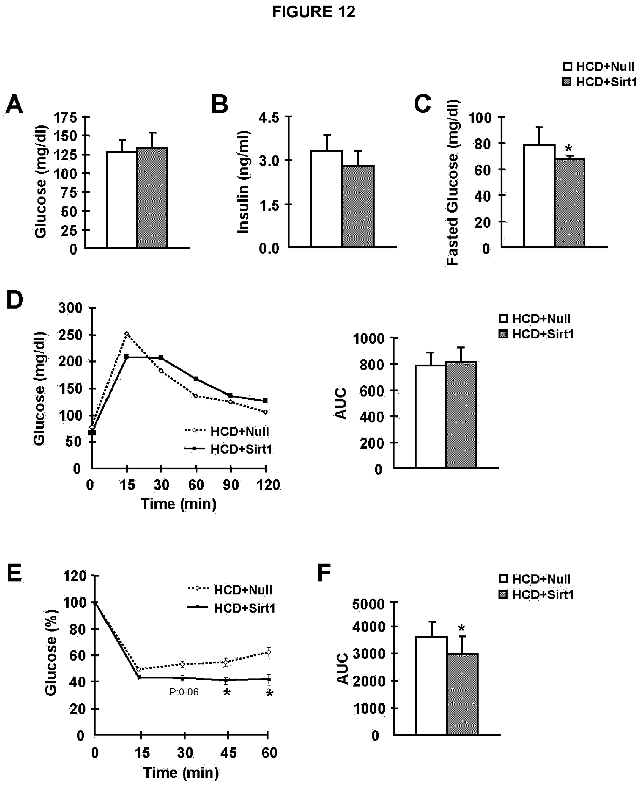

**FIGURE 12****. Sirt1 mice are protected against insulin resistance induced by a HCD. (A-B)** Blood glucose **(A)** and insulin **(B)** levels in fed conditions are shown. **(C)** Fasted glucose levels. **(D)** Glucose tolerance was determined in fasted mice after an intraperitoneal injection of glucose (1 g/kg body weight), and blood glucose levels were measured at the indicated time points. On the right, area under the curve (AUC) of the glucose tolerance test was calculated. **(E)** Insulin sensitivity was determined after an intraperitoneal injection of insulin (0.75 units/kg body weight) as described in Example 1. Results are calculated as the percentage of initial blood glucose levels. **(F)** Area under the curve (AUC) of the insulin tolerance test was calculated. Data represent the mean±SEM of at least eight animals per group. *p< 0.05 vs. CT group.

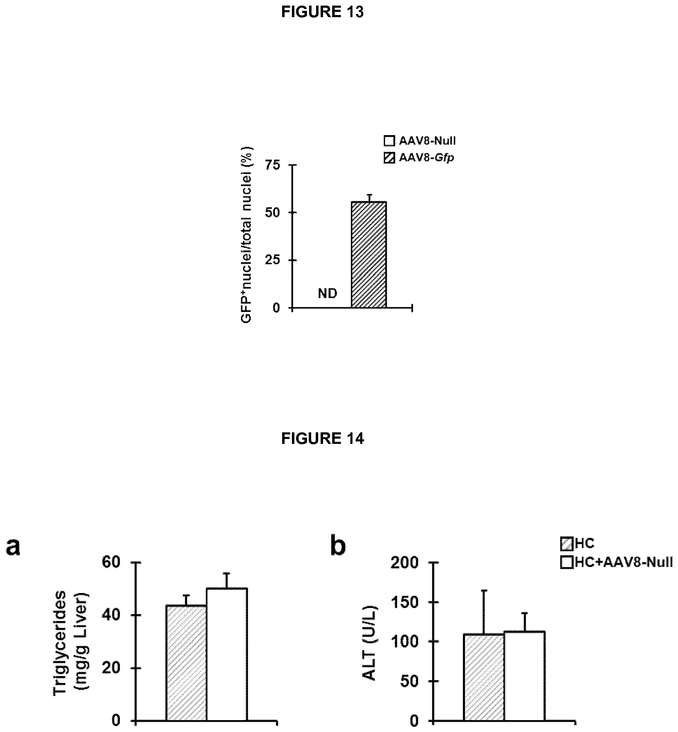

**FIGURE 13****.** Liver transduction after systemic administration of AAV8-hAAT-*Gfp*. **(a)** Immunostaining against GFP (brown) was performed in the liver of mice administered intravascularly with 5x10¹¹ vg/mice of AAV8-hAAT-Null or AAV8-hAAT-*Gfp* vectors. Original magnification x100 and x400 (insets). **(b)** Percentatge of transduced hepatocytes. All analyses were performed two weeks after AAV administration. ND, non detected.
**FIGURE 14****.** AAV8-Null injection does not affect NAFLD development. **(a)** Liver triglyceride content and **(b)** serum alanine transaminase (ALT) levels in non-treated and AAV8-Null-treated mice fed a high carbohydrate (HC) diet. All analyses were performed after 15 weeks on HC diet. Data represent the mean ± SEM of at least 4 animals per group.

### DETAILED DESCRIPTION OF THE INVENTION

The authors of the present invention have observed that the administration of viral vectors encoding and overexpressing Sirt1 under the control of a liver-specific promoter to mice fed *ad libitum* with a high carbohydrate diet (CH) to induce NAFLD, increases the expression of several genes related with β-oxidation activity that counteract the diet-induced triglyceride accumulation in the liver and, as a consequence, also reduces the hepatic inflammation induced by a high carbohydrate diet. Although Sirt1 is only overexpressed in the liver, some beneficial effects are also observed in white adipose tissue and skeletal muscle and finally in an improved whole body insulin sensitivity. Therefore, these findings suggest that long-term overexpression of Sirt1 gene transferred with AAV vectors to the liver, may be a potential preventive and/or therapeutic option for NAFLD patients.

### VIRAL CONSTRUCT

The authors of the present invention have observed that the overexpression of Sirt1 in the liver, using a viral vector wherein the sequence encoding Sirt1 is under the control of a liver-specific promoter, results in an increased expression of several genes involved in β-oxidation activity that counteract the diet-induced hepatic triglyceride accumulation and, as a consequence, reduce liver inflammation and led to the improvement of whole body metabolism. Thus, in a first aspect, the disclosure relates to a viral construct comprising a nucleotide sequence encoding Sirt1 or a functionally equivalent variant thereof which is operably linked to a liver-specific promoter.

As used herein, the term "viral construct" refers to a recombinant viral construct (i.e., viral DNA) that comprises one or more heterologous nucleotide sequences. Preferably, all other structural and non-structural coding sequences are not present in the viral vector since they can be provided in trans by a vector, such as a plasmid, or by stably integrating the sequences into a cell line. The vectors may be utilized for the purpose of transferring DNA into cells either *in vitro, in vivo* or *ex vivo.* The preferably viral constructs suitable for use in the present invention are adeno-associated viral (AAV) vectors.

The term "nucleotide sequence", is used herein interchangeably with "polynucleotide", and relates to any polymeric form of nucleotides of any length and composed of ribonucleotides and/or deoxyribonucleotides. The term includes both single-stranded and double-stranded polynucleotides as well as modified polynucleotides (methylated, protected and the like).

The term "Sirt1", as used herein, is used interchangeably with the term "Sirtuin 1". Sirt1 belongs to a family of protein deacetylases and ADP-ribosyltransferases which are involved in different pathways such as cellular metabolism, stress response, lifespan and caloric restriction. Suitable Sirt1 molecules useful for the invention include, without limitation, the homo sapiens sirtuin type 1 (Sirt1) mRNA described in NCBI under accession number AF083106) or the polypeptide described in NCBI under accession number NP_036370, corresponding to human NAD-dependent protein deacetylase sirtuin-1 isoform b.

The disclosure also contemplates the use of polynucleotides encoding Sirt1 from different animal species such as, without limitation: Mus musculus Sirtuin 1 (*Sirt1*) mRNA (GenBank Accession No. NM_001159589 or NM_019812 XM_905809) or protein (GenBank Accession No. NP_062786); Sus scrofa Sirtuin 1 (*Sirt1*) mRNA (GenBank Accession No. EU030283) or protein (GenBank Accession No. NP_001139222); Bos taurus Sirtuin 1 (*Sirt1*) mRNA (GenBank Accession No. NM_001192980) or protein (GenBank Accession No. NP_001179909.1); Xenopus tropicalis Sirtuin 1 (*Sirt1*) mRNA (GenBank Accession No. NM_001142909) or protein (GenBank Accession No. NP_001136381); Canis lupus familiaris Sirtuin 1 (*Sirt1*) mRNA (GenBank Accession No. XM_546130) or protein (GenBank Accession No. XP_546130); Xenopus laevis Sirtuin 1 (*Sirt1*) mRNA (GenBank Accession No. NM_001097726.1) or protein (GenBank Accession No. NP_001091195); Macaca mulatta Sirtuin 1 (*Sirt1*) mRNA (GenBank Accession No. XM_001087854.2) or protein (GenBank Accession No. XP_001087854); Arabidopsis thaliana Sirtuin 1 (*Sirt1*) mRNA (GenBank Accession No. NM_124958.2) or protein (GenBank Accession No. NP_200387); Gallus gallus Sirtuin 1 (*Sirt1*) mRNA (GenBank Accession No. NM_001004767.1) or protein (GenBank Accession No. NP_001004767).

The skilled person will also appreciate that, as long as the length of the viral construct does not exceed the packaging size limit of the viral capsid, the viral construct of the invention may comprise part or all of the genomic sequence encoding Sirt1, in which case, the coding region of Sirt1 will be interrupted by intronic regions. The invention also contemplates viral constructs which comprise sequences encoding Sirt1 variants and fragments known in the art.

Thus, the disclosure should be construed to include DNA encoding functional equivalent variants of Sirt1. The term "functional equivalent variant", as used herein relates to any polypeptide which sequence can be obtained from the sequence of Sirt1 as defined above by means of insertion of one or more nucleotides in the sequence, the addition of one or more nucleotides in any end or inside the sequence, or the deletion of one or more nucleotides in any end or inside the sequence and which substantially preserves the biological activity of Sirt1. Methods for determining whether a variant preserves the biological activity of the native Sirt1 are widely known to the skilled person.

Variants of Sirt1 may be obtained by replacing nucleotides within the polynucleotide accounting for codon preference in the host cell that is to be used to produce the Sirt1. Such "codon optimization" can be determined via computer algorithms which incorporate codon frequency tables such as "Ecohigh. Cod" for codon preference of highly expressed bacterial genes as provided by the University of Wisconsin Package Version 9.0, Genetics Computer Group, Madison,Wis. Other useful codon frequency tables include "Celegans_ high.cod", "Celegans_low.cod", Drosophila_high.cod", "Human_ high.cod", "Maize_ high.cod", and "Yeast_high.cod".

Variants of Sirt1 may be generated by making conservative amino acid changes and testing the resulting variant in one of the functional assays described above or another functional assay known in the art. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine.

The functionally equivalent variants of Sirt1 include polypeptides which are substantially homologous to the native SIRT1. The expression "substantially homologous", as used herein, relates to any of the nucleotide sequences mentioned above when said nucleotide sequence has a degree of identity with respect to the nucleotide sequence of the invention of at least 60%, advantageously of at least 70%, preferably of at least 85%, and more preferably of at least 95%. A nucleotide sequence that is substantially homologous to the nucleotide sequence of the invention can typically be isolated from a producer organism of the polypeptide of the invention based on the information contained in said nucleotide sequence, or it is constructed based on the DNA sequence described above. The degree of identity between two polynucleotides is determined using computer algorithms and methods that are widely known for the persons skilled in the art. The identity between two amino acid sequences is preferably determined by using the BLASTN algorithm [BLAST Manual, Altschul, S., et al., NCBI NLM NIH Bethesda, Md. 20894, Altschul, S., et al., J. Mol. Biol. 215:403-410(1990)]. BLAST and BLAST 2.0 are used, with the parameters described herein, to determine percent sequence identity. Software for performing BLAST analyses is publicly available through the National Center for Biotechnology Information. This algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always 0) and N (penalty score for mismatching residues; always 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sd. USA, 1989, 89:10915) alignments (B) of 50, expectation (E) of 10, M=5, N=-4, and a comparison of both strands.

As those skilled in the art will appreciate that variants or fragments of Sirt1 can be generated using conventional techniques, such as mutagenesis, including creating discrete point mutation(s), or by truncation. For instance, mutation can give rise to variants which retain substantially the same, or merely a subset, of the biological activity of a polypeptide from which it was derived.

As used herein, the term "Packaging" refers to a series of subcellular events that result in the assembly and encapsidation of the viral construct or the viral vector. Thus, when a suitable construct or vector is introduced into a cell line under appropriate conditions, it can be assembled into a viral particle. Functions associated with packaging of viral vectors or viral construct, are described herein and in the art.

As used herein, the term "operably linked" refers to a linkage of polynucleotide (or polypeptide) elements in a functional relationship. A nucleic acid is "operably linked" when it is placed into a functional relationship with another nucleic acid sequence. For instance, a transcription regulatory sequence is operably linked to a coding sequence if it affects the transcription of the coding sequence. "Operably linked" means that the DNA sequences are typically contiguous and, where necessary to join two protein encoding regions, contiguous and in reading frame.

As used herein, the term "promoter" or "transcription regulatory sequence" refers to a nucleic acid fragment that functions to control the transcription of one or more coding sequences, and is located upstream with respect to the direction of transcription of the transcription initiation site of the coding sequence, and is structurally identified by the presence of a binding site for DNA-dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter, including e.g. attenuators or enhancers, but also silencers. A "constitutive" promoter is a promoter that is active in most tissues under most physiological and developmental conditions. An "inducible" promoter is a promoter that is physiologically or developmentally regulated, e.g. by the application of a chemical inducer. A "tissue specific" promoter is a promoter only active in specific types of tissues or cells. Thus, a tissue specific promoter, in the context of this invention, is one which is more active in one or several (for example two, three or four) particular tissues than other tissues (i.e. is capable of driving higher expression of a coding sequence to which it is operably linked in the tissue(s) for which it is specific as compared to any others). Typically, the down-stream gene in a "tissue specific" promoter is one which is active to a much higher degree in the tissue(s) for which it is specific than in any other. In this case, there may be little or substantially no activity of the promoter in any tissue other than the one(s) for which it is specific.

As defined herein, , a liver specific promoter allows an active expression of the linked gene in the liver and prevents expression in other cells or tissues. In the context of this invention, an alpha1-antitrypsin liver specific promoter allows an active expression of the linked gene in the liver and prevents expression in other cells or tissues.

Suitable liver-specific promoters of the disclosure include, without limitation, an [alpha]1-anti-trypsin (AAT) promoter, a thyroid hormone-binding globulin promoter, an alpha fetoprotein promoter, an alcohol dehydrogenase promoter, the factor VIII (FVIII) promoter, a HBV basic core promoter (BCP) and PreS2 promoter, an albumin promoter, a phosphoenol pyruvate carboxykinase (PEPCK) promoter, a thyroxin-binding globulin (TBG) promoter, an Hepatic Control Region (HCR)-ApoCII hybrid promoter, an HCR-hAAT hybrid promoter, an AAT promoter combined with the mouse albumin gene enhancer (Ealb) element, an apolipoprotein E promoter, a low density lipoprotein promoter, a pyruvate kinase promoter, a phosphenol pyruvate carboxykinase promoter, a lecithin-cholesterol acyl transferase (LCAT) promoter, an apolipoprotein H (ApoH) promoter, the transferrin promoter, a transthyretin promoter, an alpha-fibrinogen and beta-fibrinogen promoters, an alpha 1-antichymotrypsin promoter, an alpha 2-HS glycoprotein promoter, an haptoglobin promoter, a ceruloplasmin promoter, a plasminogen promoter, promoters of the complement proteins (Clq, Clr, C2, C3, C4, C5, C6, C8, C9, complement Factor I and Factor H), C3 complement activator and the [alpha]1-acid glycoprotein promoter. Additional tissue-specific promoters may be found in the Tissue-Specific Promoter Database, TiProD (Nucleic Acids Research, J4:D104-D107 (2006).

In the invention, a liver-specific promoter is an [alpha]1-anti-trypsin (AAT) promoter.

In a preferred embodiment, the liver-specific promoter is a Hepatic Control Region (HCR)-hAAT promoter. In a still more preferred embodiment, the promoter region is located between 4321-5414 bp of the SEQ ID NO:1.

The viral constructs of the invention may also comprise polyadenylation signals operably linked to the nucleic acid encoding Sirt1 or the functionally equivalent variant thereof. The term "polyadenylation signal", as used herein, relates to a nucleic acid sequence that mediates the attachment of a polyadenine stretch to the 3' terminus of the mRNA. Suitable polyadenylation signals include the SV40 early polyadenylation signal, the SV40 late polyadenylation signal, the HSV thymidine kinase polyadenylation signal, the protamine gene polyadenylation signal, the adenovirus 5 Elb polyadenylation signal, the bovine growth hormone polydenylation signal, the human variant growth hormone polyadenylation signal and the like.

In another aspect, the viral construct of the disclosure is a parvoviral construct. The term "parvovirus" as used herein encompasses the family Parvoviridae, including autonomously-replicating parvoviruses and dependoviruses. The autonomous parvoviruses include members of the genera Parvovirus, Erythrovirus, Densovirus, Iteravirus, and Contravirus. Autonomous parvoviruses include, but are not limited to, minute virus of mouse, bovine parvovirus, canine parvovirus, chicken parvovirus, feline panleukopenia virus, feline parvovirus, goose parvovirus, H1 parvovirus, muscovy duck parvovirus, B 19 virus, and any other autonomous parvovirus now known or later discovered. Other autonomous parvoviruses are known to those skilled in the art. See, e.g., Bernard N Fields et al., Virology, Vol. 2, Chapter 69 (4th ed., Lippincott-Raven Publishers).

On the other hand, and as may be deduced from the name of their genus, members of the Dependovirus are unique in that they usually require co-infection with a helper virus such as adenovirus or herpes virus for productive infection in cell culture. The genus Dependovirus includes AAV, which normally infects humans (e.g., serotypes 1, 2, 3A, 3B, 4, 5, and 6) or primates (e.g., serotypes 1 and 4), and related viruses that infect other warm-blooded animals (e.g., bovine, canine, equine, and ovine adeno-associated viruses). Further information on parvoviruses and other members of the Parvoviridae is described in Kenneth I. Berns, "Parvoviridae: The Viruses and Their Replication," Chapter 69 in Fields Virology (3d Ed. 1996).

In the invention, the parvoviral construct is an adeno-associated virus (AAV). As used herein, the term "adeno-associated virus" (AAV) includes any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. In particular, the invention may be carried out using to AAV serotype 1 (AAV1), AAV2, AAV3 (including types 3A and 3B), AAV4, AAV5, AAV6, AAV7, AAV8, AAV9, AAV10, AAV11, avian AAV, bovine AAV, canine AAV, equine AAV, ovine AAV, and any other AAV now known or later discovered. See, e.g., Fields et al., Virology, volume 2, chapter 69 (4th ed., Lippincott-Raven Publishers). Preferred parvoviruses in the present invention are selected from AAV5, AAV7, AAV8 or AAV9. Recently, a number of putative new AAV serotypes and clades have been identified (see, e.g., Gao et al., (2004) J. Virology 78:6381-6388 ; Moris et al., (2004) Virology 33-:375-383 ; and Table 1). The genomic sequences of the various serotypes of AAV and the autonomous parvoviruses, as well as the sequences of the terminal repeats, Rep proteins, and capsid subunits are known in the art, by way of example, Srivistava et al., (1983) J. Virology 45:555 ; Chiorini et al., (1998) J. Virology 71:6823 ; Chiorini et a1,, (1999) J. Virology 73:1309 ; Bantel-Schaal et al., (1999) J. Virology 73:939 ; Xiao et al., (1999) J. Virology 73:3994 ; Muramatsu et al., (1996) Virology 221: 208 ; Shade et al., (1986) J. Virol. 58:921 ; Gao et al., (2002) Proc. Nat. Acad. Sci. USA 99:11854 ; Moris et al., (2004) Virology 33-: 375-383 ; international patent publications WO 00/28061 , WO 99/61601 , WO 98/11244 ; and U.S. Patent No. 6,156,303.

For convenience, present invention is further exemplified and described herein by reference to AAV. It is however understood that the disclosure is not limited to AAV but may equally be applied to other parvoviruses.

The genomic organization of all known AAV serotypes is very similar. The genome of AAV is a linear, single-stranded DNA molecule that is less than about 5,000 nucleotides (nt) in length. Inverted terminal repeats (ITRs) flank the unique coding nucleotide sequences for the non-structural replication (Rep) proteins and the structural (VP) proteins. The VP proteins (VP1, -2 and -3) form the capsid. The terminal 145 nt are self-complementary and are organized so that an energetically stable intramolecular duplex forming a T-shaped hairpin may be formed. These hairpin structures function as an origin for viral DNA replication, serving as primers for the cellular DNA polymerase complex. Following wtAAV infection in mammalian cells the Rep genes (i.e. Rep78 and Rep52) are expressed from the P5 promoter and the P19 promoter, respectively and both Rep proteins have a function in the replication of the viral construct. A splicing event in the Rep ORF results in the expression of actually four Rep proteins (i.e. Rep78, Rep68, Rep52 and Rep40). However, it has been shown that the unspliced mRNA, encoding Rep78 and Rep52 proteins, in mammalian cells are sufficient for AAV vector production. Also in insect cells the Rep78 and Rep52 proteins suffice for AAV vector production.

A "recombinant parvoviral or AAV construct" (or "rAAV construct") herein refers to a vector or construct comprising one or more polynucleotide sequences of interest, genes of interest or "transgenes" that are flanked by at least one parvoviral or AAV inverted terminal repeat sequences (ITRs). Such rAAV vectors can be replicated and packaged into infectious viral particles when present in an insect host cell that is expressing AAV rep and cap gene products (i.e. AAV Rep and Cap proteins). When a rAAV vector is incorporated into a larger nucleic acid construct (e.g. in a chromosome or in another vector such as a plasmid or baculovirus used for cloning or transfection), then the rAAV vector is typically referred to as a "pro-vector" which can be "rescued" by replication and encapsidation in the presence of AAV packaging functions and necessary helper functions.

Thus, in another aspect the disclosure relates to a nucleic acid construct comprising a nucleotide sequence encoding Sirt1 or a functionally equivalent variant thereof as herein defined above, wherein the nucleic acid construct is a recombinant parvoviral or AAV vector and thus comprises at least one parvoviral or AAV ITR. Preferably, in the nucleic acid construct the nucleotide sequence encoding the Sirt1 or a functionally equivalent variant thereof is flanked by parvoviral or AAV ITRs on either side. Any AAV ITR may be used in the constructs of the invention, including ITRs from AAV1, AAV2, AAV4, AAV5, AAV7, AAV9 and/or AAV8. ITRs of AAV2 or AAV8 are most preferred.

AAV sequences that may be used in the present invention can be derived from the genome of any AAV serotype. Generally, the AAV serotypes have genomic sequences of significant homology at the amino acid and the nucleic acid levels, provide an identical set of genetic functions, produce virions which are essentially physically and functionally equivalent, and replicate and assemble by practically identical mechanisms. For the genomic sequence of the various AAV serotypes and an overview of the genomic similarities see e.g. GenBank Accession number U89790; GenBank Accession number J01901; GenBank Accession number AF043303; GenBank Accession number AF085716; GenBank Accession number NC_006152; GenBank Accession number Y18065; GenBank Accession number NC_006260; GenBank Accession number NC_006261; Chlorini et al. (1997, J. Vir. 71: 6823-33); Srivastava et al. (1983, J. Vir. 45:555-64); Chlorini et al. (1999, J. Vir. 73:1309-1319); Rutledge et al. (1998, J. Vir. 72:309-319); and Wu et al. (2000, J. Vir. 74: 8635-47). AAV serotypes 1, 2, 3, 4, 5, 8 and 9 are preferred source of AAV nucleotide sequences for use in the context of the present invention. Preferably the AAV ITR sequences for use in the context of the present invention are derived from AAV1, AAV2, AAV9 and/or AAV8.

Although it is preferred that the nucleic acid sequences encoding the capsid genes are provided in trans by the cell or by a second vector, the invention also contemplates AAV constructs which further comprise a sequence encoding one or more capsid proteins which package the above mentioned polynucleotide sequence. The sequences coding for the VP1, VP2, and VP3 capsid proteins for use in the context of the present invention may however be taken from any of the known 42 serotypes, more preferably from AAV1, AAV2, AAV3, AAV4, AAV5, AAV6, AAV7, AAV8 or AAV9 or newly developed AAV-like particles obtained by e.g. capsid shuffling techniques and AAV capsid libraries. When the sequences encoding the capsid proteins derive from a different AAV serotype as the ITRs, the AAV construct is known as a "hybrid" parvovirus genome (i.e., in which the AAV capsid and the AAV terminal repeat(s) are from different AAV) as described in international patent publication WO 00/28004 and Chao et al., (2000) Molecular Therapy 2:619 . As described herein, the rAAV vector can be any suitable rAAV vector now known or later discovered. Alternatively, the sequences encoding the capsid genes may be provided in trans by co-transfecting into the cell a polynucleotide encoding said capsid proteins. In a preferred embodiment, the viral vector comprises ITRs from AAV1, AAV2 and/or AAV8 and one or more or all capsid genes from AAV1, AAV2, AAV5, AAV6 or AAV8.

If the viral vector comprises sequences encoding the capsid proteins, these may be modified so as to comprise an exogenous targeting sequence. Suitable exogenous targeting sequences are described in detail below in the context of the virions of the invention.

Optionally, the AAV constructs of the invention may comprise additional sequences coding for the Rep proteins. The Rep (Rep78/68 and Rep52/40) coding sequences are preferably derived from AAV1, AAV2, and/or AAV4. AAV Rep and ITR sequences are particularly conserved among most serotypes. US2003148506 reports that AAV Rep and ITR sequences also efficiently cross-complement other AAV Rep and ITR sequences in insect cells.

Typically, the AAV construct of the invention comprises, in addition to the expression cassette comprising the liver-specific promoter and the sequence encoding Sirt1 or the functionally equivalent variant thereof, one or more of the following elements:
Inverted terminal repeats
Non resolvable terminal repeats
Sequences encoding capsid genes
Stuffer sequences to complete the minimal packageable genome size

The inverted terminal repeats (ITR) are typically present in at least two copies of the AAV vector, typically flanking the expression cassette containing the heterologous sequence. The ITRs typically will be at the 5' and 3' ends of the heterologous nucleotide sequence(s), but need not be contiguous thereto. The terminal repeats can be the same or different from each other. The term "terminal repeat" includes any viral terminal repeat and/or partially or completely synthetic sequences that form hairpin structures and function as an inverted terminal repeat, such as the "double-D sequence" as described in United States Patent No. 5,478,745 to Samulski et al. An "AAV terminal repeat" may be from any AAV, including but not limited to serotypes 1, 2, 3, 4, 5, 6, 7, 8, 9, 10, 11 or 12 or any other AAV now known or later discovered. The AAV terminal repeat need not have a wild- type sequence (e.g., a wild-type sequence may be altered by insertion, deletion, truncation or missense mutations), as long as the terminal repeat mediates the desired functions, e.g., replication, nicking, virus packaging, integration, and/or provirus rescue, and the like. The vector construct can comprise one or more (e.g., two) AAV terminal repeats, which may be the same or different. Further, the one or more AAV terminal repeats can be from the same AAV serotype as the AAV capsid, or can be different. In particular embodiments, the vector construct comprises an AAV1, AAV2, AAV3, AAV4, AAV5, AAV6, AAV7, AAV8, AAV9, AAV10, AAV11 and/or AAV12 terminal repeat, in particularfrom AAV2, AAV8 and/or AAV9. In a preferred embodiment, the ITRs may derive from AAV2 and may be located between 356-507 bp (3'ÍTR) and between 4116-4256 bp (5'ÍTR) of the SEQ ID NO:1.

The AAV constructs of the invention may also contain non-resolvable terminal repeats. The expression "non-resolvable terminal repeat", as used herein, relates to terminal repeats which are not recognized by and resolved (i.e., "nicked") by the AAV Rep proteins, such that resolution of the terminal repeat is substantially reduced (e.g., by at least about 50%, 60%, 70%, 80%. 90%, 95%, 98% or greater as compared with a resolvable terminal repeat) or eliminated. Such non-resolvable terminal repeats may be naturally-occurring terminal repeat sequences (including altered forms thereof) and, for example, can be derived from a parvovirus, including an AAV, or can be from another virus or, as a further alternative, can be partially or completely synthetic. The non-resolvable terminal repeat may be a non-AAV viral sequence that is not recognized by the AAV Rep proteins, or it can be an AAV terminal repeat that has been modified (e.g., by insertion, substitution and/or deletion) so that it is no longer recognized by the AAV Rep proteins. Further, a non- resolvable terminal repeat can be any terminal repeat that is non-resolvable under the conditions used to produce the virus vector. Further, an AAV terminal repeat can be modified so that resolution by the AAV Rep proteins is substantially reduced or eliminated. The non-resolvable terminal repeat can be any inverted repeat sequence that forms a hairpin structure and cannot be nicked by the AAV Rep proteins.

Parvoviral ITR nucleotide sequences are typically palindromic sequence, comprising mostly complementary, symmetrically arranged sequences also referred to as "A," "B," and "C" regions. The ITR functions as a origin of replication, a site having a "cis" role in replication, i.e., being a recognition site for trans acting replication proteins such as e.g. Rep 78 (or Rep68) which recognize the palindrome and specific sequences internal to the palindrome. One exception to the symmetry of the ITR sequence is the "D" region of the ITR. It is unique (not having a complement within one ITR). Nicking of single-stranded DNA occurs at the junction between the A and D regions. It is the region where new DNA synthesis initiates. The D region normally sits to one side of the palindrome and provides directionality to the nucleic acid replication step. A parvovirus replicating in a mammalian cell typically has two ITR sequences. It is, however, possible to engineer an ITR so that binding sites are on both strands of the A regions and D regions are located symmetrically, one on each side of the palindrome. On a double-stranded circular DNA template (e.g., a plasmid), the Rep78- or Rep68- assisted nucleic acid replication then proceeds in both directions and a single ITR suffices for parvoviral replication of a circular vector. Thus, one ITR nucleotide sequence can be used in the context of the present invention. Preferably, however, two or another even number of regular ITRs are used. Most preferably, two ITR sequences are used. Accordingly, in the invention, at least one ITR may be used, i.e. one ITR may be used, although more typically two ITRs will be used.

An AAV construct of the disclosure comprises a polynucleotide which contains an expression cassette formed by the liver-specific promoter, the sequence encoding Sirt1 or a functionally equivalent variant thereof and the polyadenylation signal, wherein said expression cassette is flanked by AAV ITRs. In a still more preferred aspect, the liver-specific promoter is the alpha 1-antitrypsin promoter region.

The viral construct of the disclosure can be a single-stranded parvovirus vector, such as an AAV vector. In a preferred embodiment, the AAV vector is a single-stranded AAV (ssAAV). The expression "single-stranded parvovirus vector", as used herein, relates to a single-stranded polynucleotide (typically, DNA) packaged within an AAV capsid. As used herein, the term "single-stranded", when used in reference to a nucleic acid molecule, refers to a nucleic acid molecule which is not hybridized to another nucleic acid molecule and has no regions which will hybridize intramolecularly either under physiological conditions or stringent conditions. This is in contrast to double-stranded target which exists as two strands of nucleic acid which are held together by inter-strand base pairing interactions. The single stranded nucleic acid molecule is either sense strand or antisense strand, as both strands are equally infectious.

Typically, the rAAV vector construct only retains the minimal terminal repeat sequence(s) (each 145 bases) so as to maximize the size of the transgene that can be efficiently packaged by the vector.

The rAAV vector of the invention may also comprise a transcription termination signal. While any transcription termination signal may be included in the vector of the invention, preferably, the transcription termination signal is the SV40 transcription termination signal.

Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having about 75-99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.

### RECOMBINANT VIRIONS

In another aspect, the invention relates to virions comprising the viral construct of the invention packaged inside a capsid and, optionally a lipidic envelope surrounding said capsid.

The term "virion", "recombinant virus particle" and "viral vector" are used herein interchangeably and relate to an infectious, replication-defective virus particle comprising the viral construct of the invention packaged within a capsid and, as the case may be, a lipidic envelope surrounding the capsid.

In another embodiment, if the virion is obtained by packaging of an AAV vector or a viral construct of the invention, the virion of the invention is a "recombinant AAV virion". The term, "recombinant AAV virion" or "rAAV virion", as used herein, refers to an infectious, replication-defective virus composed of an AAV protein shell encapsidating a heterologous nucleotide sequence of interest that is flanked on both sides by AAV ITRs and one or more Rep proteins.

The term "Cap protein", as used herein, refers to a polypeptide having at least one functional activity of a native AAV Cap protein (e.g., VP1, VP2, VP3). Examples of functional activities of Cap proteins (e.g., VP1, VP2, VP3) include the ability to induce formation of a capsid, facilitate accumulation of single-stranded DNA, facilitate AAV DNA packaging into capsids (i.e., encapsidation), bind to cellular receptors, and facilitate entry of the virion into host cells. In a preferred embodiment, the polynucleotide sequence encoding the cap gene corresponds to the AAV8 cap gene. The shell of an AAV virion shows icosahedral symmetry and usually contains a major Cap protein, usually the smallest of the Cap protein and one or two minor Cap protein or proteins.

The term "Rep protein", as used herein, refers to a polypeptide having at least one functional activity of a native AAV Rep protein (e.g., Rep 40, 52, 68, 78). A "functional activity" of a Rep protein (e.g., Rep 40, 52, 68, 78) is any activity associated with the physiological function of the protein, including facilitating replication of DNA through recognition, binding and nicking of the AAV origin of DNA replication as well as DNA helicase activity. Additional functions include modulation of transcription from AAV (or other heterologous) promoters and site-specific integration of AAV DNA into a host chromosome. In a preferred embodiment, the polynucleotide sequence encoding the rep gene corresponds to the AAV1 rep gene.
The skilled person will understand that the AAV virions of the invention may comprise capsid proteins from any AAV serotype. However, due to the different tropism of the known AAV serotypes for different cells, the AAV virions will contain a capsid protein which is more adequate for delivery to the liver cells. For transduction of liver cells rAAV virions with AAV1, AAV8 and AAV5 capsid proteins are preferred (Nathwani et al., 2007, Blood 109: 1414-1421; Kitajima et al., 2006, Atherosclerosis 186:65-73).

Additionally, the AAV constructs of the invention include also AAV constructs which have been prepared by DNA shuffling as described by Stemmer, W. P. C., (Nature 270:389-391, 1994); Schmidt-Dannert et al., (Nat. Biotech. 18:750-753, 2000) and Oreneis et al., (Nat. Struct. Biol. 9:238-242, 2001). DNA or gene shuffling involves the creation of random fragments of members of a gene family and their recombination to yield many new combinations. To shuffle AAV capsid genes, several parameters are to be considered, including: involvement of the three capsid proteins VP1, VP2, and VP3 and different degrees of homologies between 8 serotypes. To increase the likelihood of obtaining a viable rcAAV vector with a cell- or tissue-specific tropism, for example, a shuffling protocol yielding a high diversity and large number of permutations is preferred. Modified "AAV" sequences also can be used in the context of the present invention, e.g. for the production of rAAV vectors in insect cells. Such modified sequences e.g. include sequences having at least about 70%, at least about 75%, at least about 80%, at least about 85%, at least about 90%, at least about 95%, or more nucleotide and/or amino acid sequence identity (e.g., a sequence having about 75-99% nucleotide sequence identity) to an AAV1, AAV2, AAV3, AAV4, AAV5, AAV6, AAV7, AAV8 or AAV9 ITR, Rep, or VP can be used in place of wild-type AAV ITR, Rep, or VP sequences.
The Rep (Rep78/68 and Rep52/40) coding sequences may be from any AAV serotype, but preferably derived from AAV1, AAV2, and/or AAV4. The sequences coding for the VP1, VP2, and VP3 capsid proteins for use in the context of the present invention may however be taken from any of the known 42 serotypes, more preferably from AAV1, AAV2, AAV5, AAV6 or AAV8.

The disclosure also contemplates virions comprising a capsid and a recombinant viral construct, wherein an exogenous targeting sequence has been inserted or substituted into the native capsid. The virion is preferably targeted (i.e., directed to a particular cell type or types) by the substitution or insertion of the exogenous targeting sequence into the capsid. Alternatively stated, the exogenous targeting sequence preferably confers an altered tropism upon the virion. As yet a further alternative statement, the targeting sequence increases the efficiency of delivery of the targeted vector to a cell.
The exogenous targeting sequence(s) may replace or substitute part or all of a capsid subunit, alternatively, more than one capsid subunit. As a further alternative, more than one exogenous targeting sequence (e.g., two, three, four, five or more sequences) may be introduced into the virion capsid. In alternative embodiments, insertions and substitutions within the minor capsid subunits (e.g., Vp1 and Vp2 of AAV) are preferred. For AAV capsids, insertions or substitutions in Vp2 or Vp3 are also preferred.

In more preferred aspects, the exogenous targeting sequence may be any amino acid sequence encoding a peptide or protein, which is inserted or substituted into the virion capsid to alter the tropism of the virion. The native virion tropism may be reduced or abolished by insertion or substitution of the amino acid sequence. Alternatively, the insertion or substitution of the exogenous amino acid sequence may target the virion to a particular cell type(s). The exogenous targeting sequence may be any amino acid sequence encoding a protein or peptide that alters the tropism of the virion. In particular embodiments, the targeting peptide or protein may be naturally occurring or, alternately, completely or partially synthetic. Exemplary peptides and proteins include ligands and other peptides that bind to cell surface receptors present in liver cells include ligands capable of binding the Sr-B1 receptor for Apoliprotein E, galactose- and lactose-specific lectins, low density lipoprotein receptor ligands, asialoglycoprotein (galactose-terminal) ligands and the like.

Alternatively, the exogenous targeting sequence may be an antibody or an antigen-recognizing moiety thereof. The term "antibody" as used herein refers to all types of immunoglobulins, including IgG, IgM, IgA, IgD, and IgE. The antibodies may be monoclonal or polyclonal and may be of any species of origin, including (for example) mouse, rat, rabbit, horse, or human, or may be chimeric antibodies. Also encompassed by the term "antibody" are bispecific or "bridging" antibodies as known by those skilled in the art. Antibody fragments include, for example, Fab, F(ab')2, and Fc fragments, and the corresponding fragments obtained from antibodies other than IgG. Such fragments may be produced by known techniques. The exogenous amino acid sequence inserted into the virion capsid may be one that facilitates purification or detection of the virion. It is not necessary that the exogenous amino acid sequence also alters the virion of the modified parvovirus. For example, the exogenous amino acid sequence may include a poly-histidine sequence that is useful for purifying the virion over a nickel column, as is known to those skilled in the art or an antigenic peptide or protein that may be employed to purify the virion by standard immunopurification techniques. Alternatively, the amino acid sequence may encode a receptor ligand or any other peptide or protein that may be used to purify the modified virion by affinity purification or any other techniques known in the art (e.g., purification techniques based on differential size, density, charge, or isoelectric point, ion-exchange chromatography, or peptide chromatography).

It is preferred to insert the exogenous amino acid sequence within the parvovirus minor Cap subunits, e.g., within the AAV Vp1 and Vp2 subunits. Alternately, insertions in Vp2 or Vp3 are also preferred.

In a preferred aspect, the virions of the disclosure comprise a viral construct which comprises a nucleotide sequence encoding Sirt1 or a functionally equivalent variant thereof which is operably linked to a liver-specific promoter. In the invention, the liver specific promoter comprises the alpha1-antitrypsin promoter. In another embodiment, the Sirt1 corresponds to a human Sirt1.

### THERAPEUTIC METHODS

The authors of the present invention have observed that the administration of the viral construct or the virions of the invention to animals fed with a high carbohydrate diet, which induce NAFLD, leads to an increased expression of several genes related to β-oxidation activity (*mitochondrial uncoupling protein 2 (Ucp2), peroxisome proliferator activated receptor δ (Ppard)* and *pyruvate deshydrogenase kinase isoenzyme 4 (Pdk4)*) that counteract the diet-induced hepatic triglyceride accumulation and, as a consequence, reduce liver inflammation. Moreover, high-carbohydrate diet induced a leptin resistant state in null mice that was attenuated in Sirt1-overexpressing mice. Finally, all these effects led to the improvement of whole body metabolism in Sirt1 mice. Thus, in another aspect, the invention relates to the viral construct of the invention for use as a medicament. In another aspect, the invention pertains to a pharmaceutical composition comprising the viral construct as herein defined above. The pharmaceutical composition further preferably comprises a pharmaceutically acceptable carrier. Any suitable pharmaceutically acceptable carrier or excipient can be used in the present compositions (See e.g., Remington: The Science and Practice of Pharmacy, Alfonso R. Gennaro (Editor) Mack Publishing Company, April 1997). Preferred pharmaceutical forms would be in combination with sterile saline, dextrose solution, or buffered solution, or other pharmaceutically acceptable sterile fluids. Alternatively, a solid carrier may be used such as, for example, microcarrier beads.

In yet another aspect, the disclosure relates to a method for the treatment and/or prevention or prophylaxis of NAFLD and related diseases, selected from insulin resistance, diabetes mellitus, metabolic syndrome and hyperlipidemia, comprising the administration to a subject in need thereof of the viral construct or the virion or the pharmaceutical composition of the invention.

In another aspect, the disclosure relates to the use of the viral construct or the virion according to the invention for the manufacture of a medicament for the prevention and/or treatment of NAFLD and related diseases selected from insulin resistance, diabetes mellitus, metabolic syndrome and hyperlipidemia.

In yet another aspect, the invention relates to the viral construct or the virion or the pharmaceutical composition according to the invention for use, in the treatment and/or prevention of NAFLD and related diseases, selecting from insulin resistance, diabetes mellitus, metabolic syndrome and hyperlipidemia.

The term "Non-alcoholic fatty liver disease" (NAFLD), as used herein, relates to a condition occurring when fat is deposited (steatosis) in the liver not due to excessive alcohol use. It is related to insulin resistance and the metabolic syndrome and may respond to treatments originally developed for other insulin-resistant states (e.g. diabetes mellitus type 2) such as weight loss, metformin and thiazolidinediones. Non-alcoholic steatohepatitis (NASH) is the most extreme form of NAFLD, and is regarded as a major cause of cirrhosis of the liver of unknown cause. The therapeutic method of the disclosure is suitable for the treatment of NAFLD, including NASH and related diseases. The term "NAFLD and related diseases" refers to insulin resistance and diabetes mellitus, metabolic syndrome, hyperlipidemia, etc.

The dose of virions, viral construct or pharmaceutical composition and the time of administration of such compositions will be within the purview of the skilled artisan having benefit of the present teachings. In fact, the inventors contemplate that the administration of therapeutically-effective amounts of the viral construct or the virions of the invention may be achieved by a single administration, such as for example, a single injection of sufficient numbers of infectious particles to provide therapeutic benefit to the patient undergoing such treatment. Alternatively, in some circumstances, it may be desirable to provide multiple, or successive administrations of the virion compositions, either over a relatively short, or a relatively prolonged period of time, as may be determined by the medical practitioner overseeing the administration of such compositions. For example, the number of infectious particles administered to a mammal may be on the order of about 10⁷, 10⁸, 10⁹, 10¹⁰, 10¹¹, 10¹², 10¹³, or even higher, infectious particles/ml given either as a single dose, or divided into two or more administrations as may be required to achieve therapy of the particular disease or disorder being treated. In fact, in certain embodiments, it may be desirable to administer two or more different virion vector compositions, either alone, or in combination with one or more other therapeutic drugs to achieve the desired effects of a particular therapy regimen. In most virion-based gene therapy regimens, the inventors believe that the use of a liver-specific promoter to control the expression of Sirt1 or of the functional equivalent variant thereof will result in a lower titer of infectious particles required when using the virions according to the invention than compared to conventional gene therapy protocols.

The term "therapeutically effective amount" or "therapeutically effective dose" refers to the amount of the compound, viral construct, virion, pharmaceutical composition, etc as disclosed in the present invention, that will elicit the biological or medical response of a tissue, system, animal or human that is being sought by the researcher, veterinarian, medical doctor or other clinician. These terms include that amount of a compound, viral construct, virion, pharmaceutical composition, etc as disclosed in the present invention that, when administered, is sufficient to prevent the development of, or alleviate to some extent, one or more of the symptoms of the condition or disorder being treated. The therapeutically effective amount will differ depending on the compound, viral construct, virion, pharmaceutical composition, etc as disclosed in the present invention, the disease and its severity and the age, weight, etc., of the mammal to be treated.

In particularly preferred embodiments of the invention, the nucleotide or peptidic sequence of interest (Sirt1), or the viral construct, or the virion, or the pharmaceutical composition, including the nucleotide sequence of interest (Sirt1), is delivered to the liver of the subject. Administration to the liver may be achieved by any method known in the art, including, but not limited to intravenous administration, intraportal administration, intrabiliary administration, intra-arterial administration, and direct injection into the liver parenchyma. In a preferred embodiment, the virion is administered intravenously.

### METHODS FOR THE PREVENTION AND/OR TREATMENT OF NON-ALCOHOLIC FATTY LIVER DISEASE USING PARVOVIRAL VECTORS ENCODING SIRT1.

The results provided by the authors of the present invention have shown that the administration of recombinant parvoviral virions comprising the Sirt1 coding sequence results in an increase in the expression of several genes related with β-oxidation activity that counteract the diet-induced triglyceride accumulation in the liver and, as a consequence, also reduces the hepatic inflammation induced by a high carbohydrate diet. Thus, the virions of the present invention are also suitable for preventing the development of NAFLD and related diseases, selected from insulin resistance, diabetes mellitus, metabolic syndrome and hyperlipidemia.

Thus, in another aspect, the disclosure relates to a recombinant parvovirus comprising a sequence encoding Sirt1 or a functionally equivalent variant thereof for use as a medicine, and more preferably for use in the treatment and/or prevention of NAFLD and relates diseases.

Thus, in another aspect, the invention relates to a recombinant AAV comprising a sequence encoding Sirt1 wherein the nucleotide sequence has at least 60% identity, preferably at least 70% identity, more preferably at least 85% identity, even more preferably at least 95% identity with the nucleotide sequence described in NCBI under accession number AF083106, wherein said nucleotide sequence is operably linked to an alpha1-antitrypsin liver-specific promoter and wherein said AAV is for use as a medicine, and more preferably for use in the treatment and/or prevention of NAFLD and related diseases, selected from insulin resistance, diabetes mellitus, metabolic syndrome and hyperlipidemia.

In another aspect, the disclosure relates to the use of a recombinant parvovirus comprising a sequence encoding Sirt1 or a functionally equivalent variant thereof for the preparation of a medicament for the treatment and/or prevention of NAFLD and related diseases, selected from insulin resistance, diabetes mellitus, metabolic syndrome and hyperlipidemia.

In another aspect, the disclosure relates to a method for the treatment of NAFLD and related diseases, selected from insulin resistance, diabetes mellitus, metabolic syndrome and hyperlipidemia, comprising the administration to a subject in need thereof of a therapeutically effective dose of recombinant parvovirus comprising a sequence encoding Sirt1 or a functionally equivalent variant thereof, or the viral construct, or the virion, or the pharmaceutical composition disclosed in the present invention.

The term "treatment", as used herein, refers to the act of reversing, alleviating, or inhibiting the progression of the disorder or condition to which such term applies, or one or more symptoms of such disorders or condition.

The term "prevention", as used herein, refers to the act of keeping from happening, existing, or alternatively delaying the onset or recurrence of a disease, disorder, or condition to which such term applies, or of one or more symptoms associated with a disease, disorder, or condition.

The term "therapeutically effective amount" or "therapeutically effective dose" refers to the amount of the compound, viral construct, virion, pharmaceutical composition, etc as disclosed in the present invention, that will elicit the biological or medical response of a tissue, system, animal or human that is being sought by the researcher, veterinarian, medical doctor or other clinician. These terms include that amount of a compound, viral construct, virion, pharmaceutical composition, etc as disclosed in the present invention that, when administered, is sufficient to prevent development of, or alleviate to some extent, one or more of the symptoms of the condition or disorder being treated. The therapeutically effective amount will vary depending on the compound, viral construct, virion, pharmaceutical composition, etc as disclosed in the present invention, the disease and its severity and the age, weight, etc., of the mammal to be treated.

The term "parvovirus" has also been described in detail above. In the invention, the parvovirus is an adeno-associated virus (AAV). Still more preferably, the AAV comprises a construct comprising the sequence encoding Sirt1 or the functionally equivalent variant thereof flanked by ITRs, wherein said ITRs are from AAV1, AAV2 and/or AAV4. In a still more preferred embodiment, the AAV serotype is an AAV7, AAV8 or AAV9. In a preferred embodiment the recombinant parvovirus is a single-stranded parvovirus, preferably, a single-stranded AAV.

The term "Sirt1" has been described in detail before. In a preferred embodiment, the Sirt1 corresponds to human Sirt1. The sequence encoding Sirt1 or the functionally equivalent variant thereof may be operably linked to a promoter region. Suitable promoters for use in the viral construct or in the virions for use in the therapeutic methods of the disclosure include any promoter which is able to active transcription of downstream sequences in liver cells, including constitutive promoters as well as liver specific promoters (see above).

In a preferred embodiment of the disclosure, the promoter which is operably linked to the sequence encoding Sirt1 or the functionally equivalent variant thereof is a liver specific promoter. Within the context of the invention , the liver specific promoter is the alpha1-antitrypsin promoter.

The therapeutic method of the disclosure also involves the administration of parvoviral construct or parvoviral virions encoding Sirt1 or a functionally equivalent variant thereof by transducing the cells with the virions/viral particles. The cells may be present in an organism, in which case the cells are reachable by needle injection, jet injection or particle gun. On the other hand, the cells to be transduced can also be isolated from an organism, be infected outside the organism and then be returned to the organism again. Such cells are referred to as autologous cells. Moreover, as to the organism it is also possible to use allogenic cells for the transduction. In this connection, it is favourable for these cells to belong to an HLA type corresponding to the organism. The person skilled in the art knows methods of providing cells with a certain HLA type. Preferable titer of the virion preparation is usually between 10⁷ and 10⁹ infectious viruses/ml.

The present invention finds use in both veterinary and medical applications. Suitable subjects include both avians and mammals, with mammals being preferred. The term "avian" as used herein includes, but is not limited to, chickens, ducks, geese, quail, turkeys and pheasants. The term "mammal" as used herein includes, but is not limited to, humans, bovines, ovines, caprines, equines, felines, canines, lagomorphs, etc. Human subjects are the most preferred. Human subjects include infant, juvenile and adult subjects.

Therefore, the subject matter of the present disclosure also relates to a medicament or pharmaceutical composition which contains a parvoviral particle according to the disclosure, more preferably an AAV particle. Here, the medicament may additionally contain a pharmaceutically acceptable carrier. Suitable carriers and the formulation of such medicaments are known to the person skilled in the art. Suitable carriers comprise e.g. phosphate-buffered saline solutions, water, emulsions, e.g. oil/water emulsions, wetting agents, sterile solutions, etc. The kind of carrier will depend on how the parvoviral vector packaging plasmid and/or parvoviral particle will be administered according to the invention. A suitable dosage is determined by the attending physician and depends on various factors, e.g. the patient's age, sex and weight, the severity of the disease, the route of administration, etc.

Administration of the parvovirus particles of the present invention to a human subject or an animal in need thereof could be carried out by different routes of administration known in the art. For example, routes of administration include oral, rectal, transmucosal, topical, transdermal, inhalation, parenteral (e.g., intravenous, subcutaneous, intradermal, intramuscular, and intraarticular) administration, and the like, as well as direct tissue or organ injection, alternatively, intrathecal, direct intramuscular, intraventricular, intravenous, intraperitoneal, intranasal, or intraocular injections. Injectable compositions can be prepared in conventional forms, either as liquid solutions or suspensions, solid forms suitable for solution or suspension in liquid prior to injection, or as emulsions. Alternatively, one may administer the virus in a local rather than systemic manner, for example, in a depot or sustained-release formulation.

In particularly preferred aspects of the disclosure, the nucleotide sequence of interest is delivered to the liver of the subject. Administration to the liver may be achieved by any method known in the art, including, but not limited to intravenous administration, intraportal administration, intrabiliary administration, intra-arterial administration, and direct injection into the liver parenchyma. In a preferred aspect, the virions or the viral construct or the pharmaceutical composition is administered intravenously.
Dosages of the parvovirus particles will depend upon the mode of administration, the individual subject's condition, the particular virus vector, and the gene to be delivered, and can be determined in a routine manner. Exemplary doses for achieving therapeutic effects are virus titers of at least about 10⁵, 10⁶, 10⁷, 10⁸, 10⁹, 10¹⁰, 10¹¹, 10¹², 10¹³, 10¹⁴ transducing units or more, preferably about 10⁸ - 10¹³ transducing units, yet more preferably 10¹² transducing units.

In particular aspects of the disclosure, more than one administration (e.g., two, three, four, or more administrations) may be employed to achieve therapeutic levels of gene expression. According to this aspect, and as described above, it is preferred to use parvovirus vectors having different antigenic properties for each administration to obviate the effects of neutralizing antibodies.

### METHODS FOR PREPARING RECOMBINANT AAV VIRIONS

Infectious AAV vector particles were generated in HEK293 cells cultured in roller bottles (RB), by co-transfecting each RB with 125 µg of the vector plasmid (containing the ITRs and the expression cassette) together with 125 µg of the rep/cap plasmid (expressing capsid proteins of the AAV particle and proteins necessary for virus replication), and 150 µg of the helper plasmid pWEAD expressing adenovirus helper functions by calcium phosphate coprecipitation (rep/cap and pWEAD plasmids were kindly provided by Dr. High, Children's Hospital of Philadelphia, USA). A total of 10 RB were used for each vector preparation.

Three days after transfection, cells were harvested and centrifuged at 2500g for 10 min. Cell pellet and medium were then processed separately. Cell pellet was thoroughly reconstituted in TBS (50mM TrisHCl, 150mM NaCl, 2mM MgCl2, pH 8.0). After 3 freeze/thaw cycles the lysate was centrifuged at 2500g for 30 min. Supernatant from this centrifugation was added to the medium and vector particles were precipitated by incubation with 8% of PEG 8000 (Sigma) for 15h and pelleted at 2500g for 30min. This pellet, now containing vectors from cells and medium, was thoroughly reconstituted in TBS (50mM TrisHCl, 150mM NaCl, 2mM MgCl2, pH 8.0), treated with benzonase (Merck) for 30 min at 37°C and centrifuged at 10000g for 10 min. The supernatant was loaded into 37.5ml ultra clear tubes (Beckman) containing 1.3-1.5 g/ml CsCI density step gradient, and centrifuged for 17 hours at 28.000 rpm in a SW28 rotor (Beckman). Viral bands were carefully collected using a 10 ml syringe and 18-gauge needle and transferred to a new 12.5 ml ultraclear tube, which was filled up with 1.379 g/ml CsCI solution to generate a continuous gradient. Tubes were centrifuged at 38.000 rpm in SW40Ti rotor (Beckman) for 48 hours.

Finally, the band of full particles was collected and dialyzed in PBS using 10 KDa membranes (Slide-A-Lyzer Dialysis Products, Pierce) and filtered with 0.45 µm Millipore filters. This PEG and CsCl-based purification protocol dramatically reduces empty AAV capsids and DNA and protein impurities from the viral stock thus increasing AAV purity, which ultimately results in higher transduction *in vivo* (Ayuso et al., 2010b).

Unless defined otherwise, technical and scientific terms used herein have the same meaning as commonly understood by one of ordinary skill in the art to which this invention belongs. Singleton et al., Dictionary of Microbiology and Molecular Biology 2nd ed. , J. Wiley & Sons (New York, NY 1994) ; March, Advanced Organic Chemistry Reactions, Mechanisms and Structure 4th ed. , J. Wiley & Sons (New York, NY 1992) ; and Sambrook and Russel, Molecular Cloning: A Laboratory Manual 3rd ed., Cold Spring Harbor Laboratory Press (Cold Spring Harbor, NY 2001), provide one skilled in the art with a general guide to many of the terms used in the present application.

The invention is hereby explained by the following examples which are to be construed as merely illustrative and not limitative of the scope of the invention.

### Example 1. Materials and Methods.

### Animals

C57BI/6 mice were obtained from Harlan laboratories. Mice were kept in a specific pathogen-free facility (SER-CBATEG) and maintained under a light-dark cycle of 12h. Mice were fed ad libitum with a standard (STD) (2018S Harlan Teklad, Madison, WI) or a high carbohydrate diet (CH) (D12450B Research Diets Inc., New Brunswick, NJ) during 15 weeks. The HC diet contained 70% of calories as carbohydrates and, specifically, 35% comes from sucrose, whereas STD diet contained 60% of calories as carbohydrates but only a 5% comes from sugars. When stated, mice were fasted for 16h. Animals were anesthetized and killed and tissues of interest were excised and kept at -80°C or with formalin, until analysis. Animal care and experimental procedures were approved by the Ethics Committee in Animal and Human Experimentation of the Universitat Autònoma de Barcelona (UAB).

### Recombinant AAV8 vectors.

Optimized Sirt1 murine plasmid was obtained from GeneArt (Regensburg, Germany) and cloning strategies were performed to obtain the plasmid pGG2-HRC/hAAT-OptSir1 comprising the Sirt1 transgene driven by the liver specific human alpha 1 antitrypsin (hAAT) promoter (SEQ ID No. 1). AAV8-hAAT-Sirt1 and AAV8-hAAT-GFP vectors were generated in our lab by triple transfection of human embryonic kidney 293 cells according to standard methods as disclosed by Matsushita T. et al. (Matsushita T. et al. Adeno-associated virus vectors can be efficiently produced without helper virus. Gene Ther. 1998;5:938-945). Briefly, cells were cultured to 80% confluence in ten roller bottles (850 cm2, flat; Corning, New York, NY, USA) in DMEM 10% (vol./vol.) FBS and co-transfected by calcium phosphate method with a plasmid carrying the expression cassette flanked by the AAV2 inverted terminal repeats, a helper plasmid carrying the AAVrep and cap genes (serotypes 6, 8 and 9) and a plasmid carrying the adenovirus helper functions. AAVs were purified with an optimised method based on a polyethylene glycol precipitation step and two consecutive caesium chloride (CsCI) gradients. Purified AAV vectors were dialysed against PBS, filtered and stored at -80°C. A non-coding plasmid carrying the hAAT promoter was used to produce null particles.

### In vivo administration of AAV vectors.

The AAV8 vectors were administered by tail vein injection at a dose of 5x10¹¹vg/mice in a total volume of 200µl/mice. One week after treatment feeding with a HC diet began and was maintained for four months.

### Vector genome copy number.

Total DNA was isolated with a MasterPureDNA Purification Kit (Epicentre Biotechnologies, Madison, WI) from overnight tissue digestions in proteinase K (0.2 mg/ml). The vector genome copy number in 20 ng of total genomic DNA was determined by quantitative PCR with primers and probe specific for the SV40 element in the AAV8-hAAT-Sirt1 vector. Forward primer: 5'- AGC AAT AGC ATC ACA AAT TTC ACA A -3'; reverse primer: 5'- CAG AC A TGA TAA GAT ACA TTG ATG AGT T -3'; probe: 5'- AGC ATT TTT TTC ACT GCA TTC TAG TTG TGG TTT GTC -3'. The final values were determined by comparison with a reference standard curve built from serial dilutions of a linearized plasmid bearing the hAAT- Sirt1 expression cassette used in the study supplemented with 20 ng/µl of irrelevant mouse genomic DNA.

### Quantification of liver transduction.

Three male mice were injected intravenously with 5x1011vg/mice of AAV8-hAAT-Gfp vectors and immunohistochemistry against GFP was performed two weeks later in the liver of these mice. Detection of GFP expression was performed in five random fields per section at an original magnification X20. The percentage of transduced hepatocytes was calculated by dividing the number of GFP+ nuclei by total nuclei of each liver section.

### Gene expression analysis.

For quantitative RT-PCR analysis, total RNA was extracted from different tissues using isolation reagent (Tripure®; Roche Molecular Biochemicals, Mannheim, Germany) and Rneasy Mini Kit (Qiagen, Hilden, Germany). Total RNA (1-2µg) was retrotranscribed using the Transcriptor First Strand cDNA Synthesis Kit (Roche). Real-time PCR was performed in a LightCycler® 480 II (Roche) using LightCycler® 480 SyBR Green I Master (Roche). Used oligonucleotide sequences are described in Table 1 and in the sequence listing.

**Table 1. Primers used in the PCR analysis.**

| **SEQ. ID No.** | **Gene** | **Primer** |
|---|---|---|
| 2 | 36b4 | Forward |
| 3 | 36b4 | Reverse |
| 4 | Acadl | Forward |
| 5 | Acadl | Reverse |
| 6 | Acadm | Forward |
| 7 | Acadm | Reverse |
| 8 | Acadvl | Forward |
| 9 | Acadvl | Reverse |
| 10 | Adipoq | Forward |
| 11 | Adipoq | Reverse |
| 12 | Ccl2 | Forward |
| 13 | Ccl2 | Reverse |
| 14 | Ccl5 | Forward |
| 15 | Ccl5 | Reverse |
| 16 | Cd68 | Forward |
| 17 | Cd68 | Reverse |
| 18 | Cpt2 | Forward |
| 19 | Cpt2 | Reverse |
| 20 | F4/80 | Forward |
| 21 | F4/80 | Reverse |
| 22 | Hif1a | Forward |
| 23 | Hif1a- | Reverse |
| 24 | II1b | Forward |
| 25 | II1b | Reverse |
| 26 | II10 | Forward |
| 27 | II10 | Reverse |
| 28 | Lep | Forward |
| 29 | Lep | Reverse |
| 30 | mCpt1 | Forward |
| 31 | mCpt1 | Reverse |
| 32 | Nos2 | Forward |
| 33 | Nos2 | Reverse |
| 34 | Nrf1 | Forward |
| 35 | Nrf1 | Reverse |
| 36 | Pdk4 | Forward |
| 37 | Pdk4 | Reverse |
| 38 | Ppargc1 a | Forward |
| 39 | Ppargc1 a | Reverse |
| 40 | Ppara | Forward |
| 41 | Ppara | Reverse |
| 42 | Ppard | Forward |
| 43 | Ppard | Reverse |
| 44 | Sirt3 | Forward |
| 45 | Sirt3 | Reverse |
| 46 | Sirt4 | Forward |
| 47 | Sirt4 | Reverse |
| 48 | Sirt6 | Forward |
| 49 | Sirt6 | Reverse |
| 50 | Tnfa | Forward |
| 51 | Tnfa | Reverse |
| 52 | Ucp2 | Forward |
| 53 | Ucp2 | Reverse |
| 54 | Ucp3 | Forward |
| 55 | Ucp3 | Reverse |

### Western blot analysis.

For total protein extracts, tissues were homogenized in protein lysis buffer and centrifuged at 15000g during 15 minutes at 4°C. Nuclear extracts were isolated using the Helenius method (Helenius M. et al. J Mol Cell Cardiol 1996;28:487-498). Used primary antibodies were the following: Anti-Sirt1 dilution 1:1000 (Millipore 07-131, Billerica, MA), Anti-phospho-ACC dilution 1:1000 (Ser⁷⁹) (Millipore 07-303), Anti-AMPK dilution 1:1000 (Cell Signaling 2532, Danvers, MA) and Anti-phospho-AMPK dilution 1:1000 (Thr¹⁷²) (Cell Signaling 2531), Anti-phospho-STAT3 (Ser⁷²⁷) dilution 1:1000 (Cell Signaling 9134), Anti-ERK1/2 dilution 1:1000 (Cell Signaling 9102), Anti-phospho-ERK1/2 dilution 1:1000 (Thr202/Tyr204) (Cell Signaling 9101), Anti-Acetyl-p53-Lys379 dilution 1:1000 (Cell Signalling 2570), Anti-PGC1 dilution 1:1000 (Santa Cruz H-300, Dallas,Texas), Anti-α-tubulina dilution 1:1000 (Abcam ab4074, Cambridge, U.K.), Anti-NF-κB dilution 1:1000 (BD Biosciences 610868, Franklin Lakes, NJ). The secondary antibodies used are the antirabbit IgG horseradish peroxidase-linked antibody dilution 1:20000 (Cell Signaling 7074) and antimouse IgG horseradish peroxidase-linked antibody dilution 1:10000 (Cell Signaling 7076).

### Isolation and extraction of nuclei for SIRT1 deacetylase assay.

Aliquots of liver homogenate (without protease inhibitors) were spun through 4 mL of sucrose 30%, 10 mM Tris HCI (pH 7.5), 10 mM NaCl and 3 mM MgCl2 at 1,300 g for 10 min at 4°C; the pellet was washed with cold 10 mM Tris-HCI (pH 7.5) and 10 mM NaCl. The nuclei were suspended in 200µL of extraction buffer containing 50 mM Hepes KOH (pH 7.5), 420 mM NaCl, 0.5 mM EDTA Na2, 0.1 mM EGTA, and glycerol 10%, sonicated for 30 s, and stood on ice for 30 min. After centrifugation at 20000g for 10 min, the supernatant (crude nuclear extract) was stored at -80°C until use.

### SIRT1 deacetylase assay.

SIRT1 deacetylase activity was evaluated using 50 µg of crude nuclear extract from livers of AAV8-Null and AAV8-Sirt1-treated mice. SIRT1 activity was measured using a deacetylase fluorometric assay kit (SIRT1 Activity Assay Kit, Abcam, ab156065). The fluorescence intensity at 440 nm (exc. 340 nm) was measured every 2 minuts for a total of 60 min immediately after the addition of fluorosubstrate peptide. The results are reported as relative fluorescence/µg of protein (AU).

### Immunohistochemical and morphometrical analysis.

Tissues were fixed for 24h in formalin, embedded in paraffin and sectioned. For histological analysis, sections were deparaffinised and stained with haematoxylin/eosin. For immunohistochemical detection, sections were deparaffinised and incubated overnight at 4° C with antibody against Mac-2, 1:500 dilution (Cederlane-CL8942AP) or GFP (Abcam ab6673), washed with PBS three times for 5 minutes each wash, incubated with the corresponding secondary antibodies and revealed with ABC Complex (Vector Laboratories Ltd., UK). Sections were counterstained in Mayer's haematoxylin.

### Measurement of the adipocyte area.

A morphometric study of adipocytes size was performed as described previously by Elias I et al. (Elias I. et al. Diabetes;2012;61:1801-181). 4 animals per group were used and at least 400 adipocytes per animal were analysed.

### Glucose and insulin tolerance tests.

Glucose and insulin-tolerance tests were performed as previously described (Elias I. et al. Diabetes;2012;61:1801-181). Briefly, mice were given an intraperitoneal injection of glucose or insulin, and the glucose concentration was determined in blood samples at indicated time points using a GlucometerR Elite analyzer (Bayer, Leverkusen, Germany).

### Metabolite and hormone assays.

The triglyceride content from liver and quadriceps and the serum levels of triglycerides, total cholesterol, HDL cholesterol, free fatty acids (FFAs), glycerol, insulin, leptin and adiponectin were measured as described previously (Elias I. et al. Diabetes;2012;61: 1801-181).

### Statistical analysis.

All values are expressed as the mean±SEM (standard error of the media). Differences between groups were compared by Student t test. Statistical significance was considered when p< 0.05.

### Example 2. Liver specific Sirt1 overexpression

To check the tissue-specificity of AAV8 vectors driven by the liver specific hAAT promoter, AAV8-hAAT-GFP and AAV8-hAAT-null vectors were injected intravenously to mice at a dose of 5x10¹¹ (vg/mice) **(****Fig. 1A****).** Fifteen days after injection, tissues were excised and analyzed. Immunohistochemical analysis showed GFP positive cells only in the liver of AAV8-hAAT-GFP- injected mice, whereas no positive cells for GFP were detected in epididymal WAT (eWAT) or skeletal muscle, thus corroborating the hepatic specificity of hAAT promoter **(****Fig. 1B and 1C****).** Moreover, the quantification of hepatic GFP positive cells showed a 55% of specific hepatocyte transduction **(Fig. 13a)****.** Then, AAV8-hAAT-Sirt1 (Sirt1) and AAV8-hAAT-null vectors were injected intravenously to mice at the same dose **(****Fig.2A****).** Fifteen weeks after vector delivery, the quantification of vector genomes showed values between 5,7-8,0 vg/cell in the livers of mice that received AAV8-*Sirt1* vectors.The protein levels of SIRT1 were analyzed in the liver, quadriceps and eWAT of CT and Sirt1 mice. As expected, SIRT1 protein overexpression (3-fold change, p<0.05) was only detected in the liver of the Sirt1-injected animals, showing that the AAV8-hAAT-Sirt1 vector was working properly and confirming the liver specificity of this vector **(****Fig. 2B****).**

Furthermore, the observed increase in Sirt1 protein levels was similar to that reported in situations of caloric restriction (CR), indicating that Sirt1 overexpression was mimicking physiological conditions (Cohen HY. et al. Science 2004;305:390-392).

Then we evaluated the levels of SIRT1 activity in whole liver extracts of treated mice with two different approaches. In agreement with increased SIRT1 protein expression, SIRT1 activity showed higher (2.5-fold increase) velocity of SIRT1-mediated deacetylation of p53, a known target of SIRT1, in AAV8-*Sirt1*-treated mice in comparison with AAV8-Null-treated animals **(****Fig. 2C****).** Moreover, the levels of endogenous acetylated p53 (Ac-p53^{Lys379}) were significantly reduced by approximately 50% in liver nuclear extracts from mice that received AAV8-*Sirt1* vectors **(****Fig. 2D****),** corroborating the increase in the activity of hepatic SIRT1 after AAV8-*Sirt1* injection.

### Example 3. Lower lipid accumulation in the liver of Sirt1 mice fed with a HC diet

To examine whether an increase in hepatic Sirt1 expression could protect against the development of NAFLD, mice overexpressing Sirt1 specifically in the liver were fed a high carbohydrate (HC) diet during 15 weeks. Histological analysis showed that liver fat deposition was increased in null mice fed a HC diet compared with mice fed a standard diet **(****Fig. 3A****).** The injection of AAV8-Null vectors did not affect the degree of accumulation of lipids in the liver of animals fed a HC diet **(****Fig. 14a****).** However, this lipid accumulation was highly attenuated in Sirt1 mice **(****Fig. 4A****).** Indeed, the liver triglyceride content was decreased (-43%, p<0.05) in Sirt1 mice in comparison with null mice **(****Fig. 4B****),** in parallel with a decrease in liver weight (-17%, p<0.05) **(****Fig. 4C****).** As it is well described, HC diet induces lipid accumulation in the liver through increased lipogenesis and reduced β-oxidation activities **(****Fig. 3A and 3B****).** These processes are often regulated by acetylation/deacetylation. In this context, acetylation promotes lipogenesis and reduces lipid oxidation, whereas deacetylation induces the opposite effects (Yu J. et al. Pharmacol Res;2010;62:35-41).

Thus, we examined the effects of SIRT1 overexpression on hepatic expression levels of genes related with lipid synthesis and oxidation. No differences in the mRNA levels of lipogenic genes such as sterol regulatory element binding transcription factor 1c (Srebf1) and fatty acid synthase (*Fasn*) were observed. However, a significant increase was detected in the mRNA levels of genes controlling lipid oxidation and mitochondrial biogenesis such as *Ppargc1a,* long-chain acyl-Coenzyme A dehydrogenase (*Acadl*), very long chain acyl-CoA dehydrogenase (*Acadvl*), sirtuin 3 (*Sirt3*) and sirtuin 6 (*Sirt6*) in Sirt1 mice **(****Fig. 4D****).** A slight up-regulation in carnitine palmitoyltransferase 2 (*Cpt2*) and nuclear respiratory factor 1 (*Nrf1*) mRNA levels was also observed in Sirt1 mice **(****Fig. 4D****).** Furthermore, the PPARGC1A protein levels were also increased in the liver of Sirt1 mice **(****Fig. 4E****).** Thus, these results suggest that the reduced hepatic lipid accumulation could be due to an increase in β-oxidation activity in the liver of Sirt1 mice.

### Example 4. Sirt1 mice showed less macrophage infiltration in the liver

We next examined whether Sirt1 protects against the hepatic inflammation triggered by HC diet. To this end, immunohistochemistry for the macrophage marker MAC-2 was performed. Null mice fed with HC diet showed a great number of hepatic cells positive for MAC-2 suggesting a high degree of macrophage infiltration. Nevertheless, Sirt1 mice showed less MAC-2 positive cells than Null mice **(****Fig. 5A****).** HC diet also led to a rise in the hepatic mRNA levels of the macrophage marker *F4*/*80* and proinflammatory cytokines like interleukin 1β (*II1b*) and chemokine (C-C motif) ligand 5 (*Ccl5*) **(****Fig. 6A****),** when compared with STD diet fed mice. In accordance with the reduction in MAC-2 positive cells, Sirt1 mice showed lower mRNA expression levels of macrophage markers like Cd68 and *F4*/*80* (-40% and -81%, respectively) **(****Fig. 5B****).** Other inflammatory markers such as *II1b* and *Ccl5* were also diminished in the liver of Sirt1 overexpressing mice in comparison with Null mice **(****Fig. 5B****).** As a consequence of the lower hepatic inflammation, the serum ALT levels were also decreased in Sirt1 versus CT mice **(****Fig. 5C****),** but were not affected by the injection of the AAV8-Null vector per se **(****Fig. 14b****).** Altogether, these results suggest that Sirt1 overexpression protects the liver against NAFLD-associated inflammation.

### Example 5. Reduced hypertrophy and inflammation in eWAT of Sirt1 mice

As expected, after 15 weeks of HC diet, control mice presented higher fat accumulation in adipose tissue, which was reflected by an increase in eWAT weight and by an increase in the mean area of adipocytes related to mice fed a STD diet **(****Fig. 7A-B)**. In Sirt1 mice, although body weight gain and food intake were similar to Null mice **(****Fig. 7D-E****),** a slight decrease in eWAT weight was observed, associated to a reduction (-30%, p<0.05) in the mean area of adipocytes **(****Fig. 8A-C****).** Moreover, Sirt1 mice presented changes in the frequency distribution of the area of white adipocytes, showing a reduction in the number of large-size adipocytes and an increased number of intermediate-sized adipocytes **(****Fig. 8D****).**

WAT hypertrophy is usually related to the development of inflammation and hypoxia (Elias I. et al. Diabetes;2012;61:1801-1813). In accordance and in parallel to the fat accumulation, HC diet induced an increase in expression levels of proinflammatory cytokines like inducible nitric oxide synthase 2 (*Nos2*) and tumour necrosis factor (*Tnf*) in eWAT **(****Fig. 7C****).** Furthermore, the mRNA levels of hypoxia inducible factor 1 (*Hif1a*) were also augmented in eWAT from HC-fed mice **(****Fig. 7C****).** In accordance with the reduced hypertrophy observed in Sirt1 mice, mRNA levels of proinflammatory cytokines like chemokine (C-C motif) ligand 2 (*Ccl2*) and *Nos2,* as well as *Hif1a* expression levels, were reduced in eWAT of these mice, whereas no changes were observed in antiinflammatory cytokines like interleukin 10 (*II10*) **(****Fig. 8H****).**

In parallel to the reduced hypertrophy, the expression of leptin in eWAT was lower in Sirt1 versus Null mice, and this was also reflected in a decrease in leptin serum levels **(****Fig. 8E and 8G****).** However, no differences in adiponectin expression or circulating levels were detected **(****Fig. 8F and 8G****).** Thus, all these data suggest that inflammation and hypoxia were decreased in adipose tissue from Sirt1 mice, although Sirt1 overexpression was restricted to the liver.

### Example 6. Sirt1 mice showed increased β-oxidation activity in skeletal muscle

After 15 weeks of HC diet, Sirt1 mice showed a trend to accumulate less triglycerides in skeletal muscle (-30%, p=0.09) than null mice, suggesting that β-oxidation activity could be up-regulated in this tissue **(****Fig. 9A****).** In agreement with this, the mRNA levels of key genes involved in lipid oxidation such as mitochondrial uncoupling protein 2 (*Ucp2*), peroxisome proliferator activated receptor δ (*Ppard*) and pyruvate deshydrogenase kinase isoenzyme 4 (*Pdk4*) were increased in the skeletal muscle of Sirt1 mice **(****Fig. 9B****).** One of the main enzymes controlling skeletal muscle lipid oxidation, AMPK, showed an increased ratio of activated phosphorylated-AMPK/total AMPK in Sirt1 mice versus Null mice (+50%, p<0.05) **(****Fig. 9C****).** The phosphorylation of ACC, one of the AMPK target, was also augmented in Sirt1 versus Null mice **(****Fig. 9C****).** Thus, these results indicate that β-oxidation could be increased in the skeletal muscle of Sirt1 mice.

### Example 7. Sirt1 mice were more sensitive to the leptin actions in peripheral tissues

Since fructose intake may trigger leptin resistance in the liver, which in turn may lead to a decrease in lipid oxidation (Roglans N. et al. Hepatology 2007;45:778-788; Vila L. et al. Hepatology 2008;48:1506-1516), leptin signalling was examined in Sirt1 mice. Levels of Signal Transducer and Activator of Transcription 3 (STAT3) which in normal conditions is phosphorylated and activated in response to leptin (Ceddia RB. Int J Obes (Lond) 2005;29:1175-1183), were measured in the liver of Sirt1 and Null mice. The amount of nuclear fully activated form STAT3-PSer⁷²⁷ was increased in the liver of Sirt1 mice in comparison with Null mice (+63%, p=0.06) (**Fig. 10A**). In addition, the hepatic STAT3-PSer⁷²⁷ protein levels correlated negatively with the serum leptin levels, suggesting a correlation between leptin levels and the degree of leptin resistance (p<0.01) (**Fig. 10B**). Thus, Sirt1 mice may be more leptin sensitive than Null mice fed with a HC diet. Moreover, leptin also activates the mitogen-activated protein kinase Erk1/2 by phosphorylation (Vila L. et al. Hepatology 2008;48:1506-1516). As expected, the liver of Sirt1 mice also showed a higher amount of phosphorylated ERK2 in comparison with Null mice (**Fig. 10A**). In skeletal muscle, leptin acts mainly through AMPK activation. In Sirt1 mice, the observed increase in skeletal muscle AMPK-P/total ratio **(****Fig. 9C****)** and the negative correlation between AMPK-P/total ratio and serum leptin levels (**Fig. 10C**), also suggest an improvement of leptin sensitivity.

### Example 8. Serum lipid profile and glucose homeostasis

Despite the reduction of fat accumulation in adipose and non-adipose tissue, serum triglyceride, HDL-cholesterol, LDL-cholesterol and total-cholesterol levels remained unchanged in Sirt1 compared with Null mice fed a HC diet (**Fig. 11A, 11D, 11E and 11F**). However, Sirt1 mice presented a significant increase in serum free fatty acid levels in comparison with Null mice, whereas no changes were observed in glycerol levels, suggesting differences in FFA re-esterification between Null and Sirt1 mice (**Fig. 11B and 11C**). We next examined whether hepatic Sirt1 overexpression exerts some beneficial effects on whole body glucose homeostasis. In fed conditions, glucose and insulin levels were no different between Null and Sirt1 mice (**Fig. 12A and 12B**). However, a significant reduction was observed in the fasting glucose levels of Sirt1 mice in comparison with Null mice, suggesting a reduction in the gluconeogenic process **(****Fig. 12C****).** However, no changes were observed between groups after a glucose overload, meaning that glucose tolerance was similar in Sirt1 and Null mice (**Fig. 12D**). When an intraperitoneal insulin tolerance test was performed, Sirt1 mice showed an improved insulin response, reducing glucose levels by 60% at the end of the ITT, whereas Null mice only reduced glucose levels by 40% (**Fig. 12E**). In addition, the ITT area under the curve was significantly reduced in the Sirt1 vs. Null mice (**Fig. 12F**), indicating that insulin sensitivity was improved in Sirt1 mice.

As conclusion, the present invention demonstrate that long-term AAV-mediated hepatic Sirt1 overexpression leads to increased expression of several genes related with β-oxidation activity that counteracts the HC diet-induced NAFLD and improves whole body metabolism. NAFLD is not only a cause of chronic liver disease, but also predicts the tendency to develop diabetes mellitus, hypertension and dyslipidemia. For this reason, Sirt1 overexpression in the liver thorough parvoviral vector, preferably, AAV vectors, is a novel gene therapy approach to prevent the development of NAFLD and related diseases, selected from insulin resistance, diabetes mellitus, metabolic syndrome and hyperlipidemia.

## Claims

1. A viral construct comprising a nucleotide sequence encoding Sirtuin 1 (Sirt1)
wherein the nucleotide sequence has at least 60% identity, preferably at least 70% identity, more preferably at least 85% identity, even more preferably at least 95% identity with the nucleotide sequence described in NCBI under accession number AF083106,
wherein said nucleotide sequence is operably linked to an alpha1-antitrypsin liver-specific promoter, and wherein said viral construct is an AAV.

2. A viral construct as defined in claim 1 wherein the AAV construct is an AAV2, AAV5, AAV7, AAV8 or AAV9.

3. A viral construct as defined in claim 1 wherein the AAV construct is an AAV8.

4. A viral construct as defined in any of claims 1 to 3 wherein Sirt1 corresponds to a human Sirt1.

5. A virion comprising the viral construct as defined in any of the claims 1 to 4 packaged inside a capsid and, optionally a lipidic envelope surrounding said capsid.

6. A virion according to claim 5 for use as a medicament.

7. A virion according to any of claims 5 to 6 for use in the treatment of diseases selected from: non-alcoholic fatty liver disease (NAFLD), insulin resistance, diabetes mellitus, metabolic syndrome and hyperlipidemia.

8. A virion for use according to claim 7 wherein the disease is non-alcoholic fatty liver disease (NAFLD).

9. A pharmaceutical composition comprising the viral construct as defined in any of claims 1 to 4 or the virion as defined in claim 5 and a pharmaceutically acceptable carrier.

10. A pharmaceutical composition according to claim 9 for use in the treatment of diseases selected from: non-alcoholic fatty liver disease (NAFLD), insulin resistance, diabetes mellitus, metabolic syndrome and hyperlipidemia.

11. A pharmaceutical composition for use according to claim 10 wherein the disease is non-alcoholic fatty liver disease (NAFLD).

## Patentansprüche

1. Virales Konstrukt mit einer Nukleotidsequenz, die Sirtuin 1 (Sirt1) kodiert,
wobei die Nukleotidsequenz mindestens zu 60%, vorzugsweise mindestens zu 70%, besonders bevorzugt mindestens zu 85%, ganz besonders bevorzugt mindestens zu 95% mit der Nukleotidsequenz identisch ist, die im NCBI unter der Zugangsnummer AF083106 beschrieben ist,
wobei besagte Nukleotidsequenz operativ mit einem Alpha-1-Antitrypsin-leberspezifischen Promoter verknüpft ist, und
wobei besagtes virales Konstrukt ein AAV ist.

2. Virales Konstrukt wie in Anspruch 1 definiert, wobei das AAV-Konstrukt ein AAV2, AAV5, AAV7, AAV8 oder AAV9 ist.

3. Virales Konstrukt wie in Anspruch 1 definiert, wobei das AAV-Konstrukt ein AAV8 ist.

4. Virales Konstrukt wie in einem beliebigen der Ansprüche 1 bis 3 definiert, wobei Sirt1 einem menschlichen Sirt1 entspricht.

5. Virion, das das virale Konstrukt, wie in einem beliebigen der Ansprüche 1 bis 4 definiert, umfasst, in einer Virushülle verpackt und auf Wunsch mit einer Lipidhülle um besagte Virushülle herum.

6. Virion nach Anspruch 5 zur Verwendung als Medikament.

7. Virion nach einem beliebigen der Ansprüche 5 bis 6 zur Verwendung bei der Behandlung folgender Krankheiten: nichtalkoholische Fettleberkrankheit (NAFLD), Insulinresistenz, Zuckerkrankheit, Stoffwechselsyndrom und Hyperlipidämie.

8. Virion zur Verwendung nach Anspruch 7, wobei die Krankheit nichtalkoholische Fettleberkrankheit (NAFLD) ist.

9. Pharmazeutische Zusammensetzung mit dem viralen Konstrukt wie in einem beliebigen der Ansprüche 1 bis 4 definiert oder dem Virion wie in Anspruch 5 definiert und einem pharmazeutisch akzeptablen Träger.

10. Pharmazeutische Zusammensetzung nach Anspruch 9 zur Verwendung bei der Behandlung folgender Krankheiten: nichtalkoholische Fettleberkrankheit (NAFLD), Insulinresistenz, Zuckerkrankheit, Stoffwechselsyndrom und Hyperlipidämie.

11. Pharmazeutische Zusammensetzung zur Verwendung nach Anspruch 10, wobei die Krankheit nichtalkoholische Fettleberkrankheit (NAFLD) ist.

## Revendications

1. Construction virale comprenant une séquence nucléotidique codant pour Sirtuin 1 (Sirt1)
dans laquelle la séquence nucléotidique a au moins 60 % d'identité, de préférence au moins 70 % d'identité, plus préférentiellement au moins 85 % d'identité, encore plus préférentiellement au moins 95 % d'identité avec la séquence nucléotidique décrite dans le NCBI sous le numéro d'accession AF083106,
dans laquelle ladite séquence nucléotidique est liée de manière opérationnelle à un promoteur de l'alpha-1 antitrypsine spécifique du foie, et
dans laquelle ladite construction virale est un AAV.

2. Construction virale tel que définie dans la revendication 1 dans laquelle la construction d'AAV est un AAV2, AAV5, AAV7, AAV8 ou AAV9.

3. Construction virale tel que définie dans la revendication 1 dans laquelle la construction d'AAV est un AAV8.

4. Construction virale tel que définie dans l'une quelconque des revendications 1 à 3 dans laquelle Sirt1 correspond à une Sirt1 humaine.

5. Virion comprenant la construction virale tel que définie dans l'une quelconque des revendications 1 à 4 emballée dans une capside et, de façon facultative, une enveloppe lipidique entourant ladite capside.

6. Virion selon la revendication 5 destiné à être utilisé comme médicament.

7. Virion selon l'une quelconque des revendications 5 à 6 destiné à être utilisé dans le traitement de maladies sélectionnées parmi: stéatose hépatique non-alcoolique (NAFLD), résistance à l'insuline, diabète sucré, syndrome métabolique et hyperlipidémie.

8. Virion destiné à être utilisé selon la revendication 7 dans lequel la maladie est une stéatose hépatique non-alcoolique (NAFLD).

9. Composition pharmaceutique comprenant la construction virale tel que définie dans l'une quelconque des revendications 1 à 4 ou le virion tel que défini dans la revendication 5 et un support pharmaceutiquement acceptable.

10. Composition pharmaceutique selon la revendication 9 destinée à être utilisée dans le traitement de maladies sélectionnées parmi: stéatose hépatique non-alcoolique (NAFLD), résistance à l'insuline, diabète sucré, syndrome métabolique et hyperlipidémie.

11. Composition pharmaceutique destinée à être utilisée selon la revendication 10 dans laquelle la maladie est une stéatose hépatique non-alcoolique (NAFLD).
